# FASCICULE DE BREVET EUROPEEN

(11) **EP 3 291 905 B1**
(45) Date de publication et mention de la délivrance du brevet: **12.01.2022**
(21) Numéro de dépôt: 16727750.8
(22) Date de dépôt: 03.05.2016
(51) Int. Cl.: B01D 15/38, B01J 20/24, B01J 20/286, B01J 20/32, A61M 1/36, G01N 33/49, B01J 20/288, B01J 20/289, B01J 20/22

(54) **SUPPORT POUR LA PURIFICATION DE LIQUIDES BIOLOGIQUES**
SUBSTRAT ZUR AUFREINIGUNG VON KÖRPERFLÜSSIGKEITEN
SUBSTRATE FOR THE PURIFICATION OF BODY FLUIDS

(30) Priorité: 06.05.2015 FR 1554082
(43) Date de publication de la demande: 14.03.2018
(73) Titulaire: Elicityl, F-38920 Crolles (FR)
(72) Inventeur: BASTIDE, Ludovic, Les Adrets 38190 (FR); BONNET, Silvère, 38100 Grenoble (FR); DARBLADE, Benoît, 38920 Crolles (FR); HAVET, Stéphane, 69680 Chassieu (FR); MANDRAND, Bernard, 69100 Villeurbanne (FR); RANDRIANTSOA, Mialy, 18100 Vierzon (FR)
(74) Mandataire: Cabinet Beau de Loménie
(86) Numéro de dépôt international: PCT/FR2016/051045
(87) Numéro de publication internationale: WO 2016/177967

(56) Documents cités:
- EP-A1- 0 435 470
- EP-A2- 0 131 546
- DE-A1- 19 953 590
- FR-A1- 3 008 097
- US-A1- 2004 242 857

## Description

La présente invention concerne le domaine de la purification de liquides biologiques, notamment de produits issus du sang et, en particulier, la purification de plasmas, par l'élimination d'anticorps présents. Plus précisément, l'invention concerne un nouveau support porteur d'au moins un type d'oligosaccharide, notamment déterminant des groupes sanguins, les procédés de purification mettant en œuvre un tel support et l'utilisation d'un tel support pour stabiliser la liaison des oligosaccharides aux anticorps contenus dans un produit issu du sang.

Le système immunitaire est un mécanisme de défense de l'organisme dont la fonction est de protéger un organisme contre les agressions étrangères. La réponse dite humorale produit des anticorps dont le répertoire est propre à chaque individu. Par contre, le système immunitaire, en particulier les anticorps sécrétés par la réponse humorale, sont à l'origine de risques majeurs lors des transplantations d'organes ou de tissus animaux vers l'homme, lors de transferts interhumains (de sang, de plasma ou d'organe).

De même, de nombreuses maladies auto-immunes sont dues à la perception par l'organisme de déterminants protéiques, lipidiques ou saccharidiques de cet organisme même, comme étranger, ce qui génère une réponse immunitaire chronique. De même, lorsque l'on injecte à un individu des préparations pharmaceutiques contenant des anticorps, il est indispensable que l'antigène correspondant, s'il n'est pas la cible thérapeutique, ne soit pas présent chez le receveur pour éviter le risque de provoquer une réponse indésirable, un des exemples de cette réponse étant l'hémolyse due à des anticorps contre les déterminants antigéniques des groupes sanguins. Une préparation injectable devra donc être dépourvue de ce type d'anticorps.

Pour ce faire, la purification des anticorps anti protéiques existe depuis longtemps, mais celle des anticorps anti-saccharidiques est plus problématique, à cause de la difficulté de synthèse des antigènes (ce sont des molécules complexes résultant d'une synthèse *in vivo* multienzymatique) et de la faible constante d'affinité entre l'anticorps et l'antigène. Cette faible affinité des anticorps anti-saccharidiques conduit à mettre en présence un très large excès d'antigène par rapport aux anticorps que l'on souhaite éliminer du milieu (l'excès est d'un facteur très élevé soit 10 000 à 100 000 molécules d'antigène saccharidique pour une molécule d'anticorps, ce ratio étant diminué si l'oligosaccharide est fixé sur une phase solide, multimérisé ou polymérisé). Symétriquement, l'affinité d'un anticorps de type IgM, multimère, présentant 10 possibilités de liaison à l'antigène par molécules, est supérieure à celle des IgG qui n'ont que deux sites de liaison à l'antigène. Dans le cas des IgM, on parle alors d'avidité, pour prendre en compte l'effet synergique des multiples fonctions anticorps.

Il est donc important de développer des supports multimérisant les antigènes saccharidiques et de les adapter à la purification des IgG tout autant que des IgM.

La séparation des anticorps anti-saccharidiques, considérés comme indésirables, d'avec un liquide biologique conservant toutes ses propriétés, est une avancée thérapeutique majeure qui permet de nombreuses applications.

Lors d'une transfusion sanguine, le plasma transfusé, pour être universel, doit être débarrassé des anticorps connus ou supposés du donneur. Les transfusions de liquides biologiques, tels que le plasma, sont des actes thérapeutiques dont l'objectif est d'obtenir une régénération du système d'hémostase ou de restaurer l'hémodynamique du patient. La présence des anticorps d'un donneur de groupe non compatible est à même de mettre en danger le patient receveur, en créant un choc hémolytique. Les anticorps des groupes sanguins présents dans le plasma du donneur (anti A, anti B ou les deux en fonction du groupe sanguin) sont déterminés par des éléments saccharidiques. Dans le système de soin actuel, un patient ne reçoit que des produits sanguins identiques à son groupe sanguin ou compatibles, engendrant des coûts logistiques et de stockage importants. La proposition d'un plasma compatible pour tous les patients est une solution sécuritaire et économique.

De même, lors d'une xéno-greffe (c'est-à-dire la greffe d'un organe animal sur l'homme), les anticorps contre les déterminants de l'organe greffé doivent être éliminés pour éviter le rejet massif de l'organe. Le traitement immunosuppresseur est une solution thérapeutique au problème, mais présentant des effets secondaires pouvant engager le pronostic vital du patient et dont l'efficacité n'est pas totale. La neutralisation des xéno-anticorps du receveur par la fixation à des xéno antigènes, composés saccharidiques, constitue un apport au domaine des xéno-greffes.

Lors de maladies auto-immunes, l'excès d'auto-anticorps peut avoir des conséquences importantes. Par exemple, dans la maladie de Guillain Barré, les auto-anticorps du malade attaquent le système nerveux de celui-ci et détruisent les nerfs périphériques (parfois jusqu'à l'axone). Il a été démontré que ces anticorps se fixaient à des gangliosides (possédant un déterminant antigénique saccharidique) présents dans le système nerveux. Le motif reconnu est lui aussi saccharidique. Pouvoir éliminer ces éléments non contrôlés du système immunitaire est une piste de traitement de la maladie.

Plus précisément, dans le cas des transfusions sanguines, les éléments figurés du sang, hématies, plaquettes, lymphocytes, portent à leur surface des antigènes saccharidiques A, B ou H, qui déterminent le groupe sanguin des individus d'une espèce de primates. Les personnes du groupe sanguin A ont des antigènes de groupe A, les personnes du groupe sanguin B, des antigènes de groupe B, les personnes du groupe sanguin AB ont les deux types d'antigènes. Les personnes du groupe sanguin O portent l'antigène H, dont l'enchaînement oligosaccharidique se retrouve dans les antigènes de groupe A et B. Les antigènes A et B présentent un monosaccharide supplémentaire. Les antigènes de groupe A et les antigènes de groupe B sont, par ailleurs, portés par différents agents infectieux non pathogènes, bactéries essentiellement. La mise en contact très précoce avec ces agents infectieux entraîne une réponse immune contre le déterminant A ou B que l'individu ne porte pas. Ainsi, les individus de groupe sanguin A montrent une réponse plasmatique en générant des anticorps anti-déterminant B (également nommés anti-B) et les individus de groupe B, une réponse plasmatique en générant des anticorps anti-déterminant A (également nommés anti-A), les individus AB n'ont pas d'anticorps et ceux du groupe O des anticorps anti A et anti B. Les anticorps des groupes sanguins sont essentiellement du type IgG et IgM.

Les prélèvements plasmatiques par plasmaphérèse ou à partir de sang total ne sont utilisables pour traiter des patients, qu'après groupage sanguin et plasmatique des patients, ce qui représente un risque d'erreur, une perte de temps et une source de complexité logistique considérable.

Pour circonvenir cet inconvénient, plusieurs solutions ont été proposées. Le mélange de plasmas en proportions définies permet de diluer les concentrations d'anticorps et d'apporter un mélange d'antigènes et d'anticorps qui se neutralisent mutuellement (EP 0 896 824). Toutefois, cette solution reste très partielle, les mélanges efficaces ne correspondant pas à la fréquence des groupes souhaités dans les populations de donneurs. Cette solution est également risquée puisqu'elle laisse dans les plasmas traités des complexes immuns, source potentielle de réactions auto-immunes.

Plusieurs solutions proposent également d'éliminer les anticorps du plasma en utilisant des oligosaccharides antigéniques déterminants des groupes A ou B. Un des premiers à proposer une telle solution est Raymond U. Lemieux (US 4,238,473 publié en 1980). Depuis plusieurs approches utilisant notamment des techniques chromatographiques ont été proposées, mais ne donnent pas satisfaction en termes d'efficacité et/ou de coût. On peut notamment citer :
- la demande de brevet EP0 572 194 qui décrit une méthode pour éliminer les anticorps d'un produit du sang, tout en préservant les facteurs de coagulation (voir revendication 1). Pour cela, une matrice constituée d'une résine porteuse d'antigènes, qui peuvent être liés par liaison covalente, est décrite. Ces antigènes peuvent être des oligosaccharides. Aucun détail n'est donné sur les modes de liaison des antigènes à la matrice ;
- le brevet US4 664 913 qui décrit une méthode pour traiter du plasma humain, par passage au travers d'une zone immuno-adsorbante, capable de lier à la fois les anticorps anti-A et anti-B. La liaison antigène / support dans la zone immuno-adsorbante est réalisée par réaction d'une fonction acyl azide portée par t'antigène sur un support aminé. Référence est également faite dans ce document, au brevet US 3,555,143 qui décrit des particules porteuses de fonctions amine, carboxy ou hydroxyle pour la liaison à des anticorps et au brevet US 4,108,974 qui décrit des billes porteuses de carboxylate pour couplage avec des anticorps porteurs de fonctions aminé ;
- le document WO2008/136735 qui concerne un produit comprenant au moins un ligand, lié à un matériau de séparation, également nommé matrice, autorisant une liaison sélective à une biomolécule ou un clivage sélectif d'une biomolécule. Une des applications de ce produit est son utilisation pour réduire les anticorps dans un plasma ou dans un échantillon de sang total. La liaison à la matrice est envisagée *via* une fonction NH₂ située à l'extrémité du bras de liaison situé en bout du saccharide, notamment sur de l'agarose époxy-activée, de l'agarose tosyl-activée ou une matrice CNBr-activée ;
- la demande de brevet EP1165159 qui décrit une configuration particulière de colonnes pour le traitement du sang total ou du plasma sanguin, qui contient une matière de matrice réticulée à laquelle au moins un saccharide biologiquement actif est lié de manière covalente *via* au moins un espaceur spécifique. Le mode de liaison décrit utilise la réaction d'un oligosaccharide porteur à l'extrémité du bras espaceur d'une fonction -NH₂ sur du Sepharose-NHS-activé. D'autres types de support de type Sepharose époxy-activé sont cités. Aucun autre exemple de couplage n'est donné dans la description ;
- la demande de brevet WO 2011/046504 qui décrit un produit et une méthode pour réaliser un traitement ou une purification contenant au moins deux filtres ou un filtre et une centrifugation ou deux centrifugations et un composant qui contient au moins une substance capable de se lier à une substance cible. Il est indiqué que le ligand est lié de manière covalente à la matrice et que différentes méthodes choisies par l'homme du métier pourront être utilisées. Le mode de liaison détaillé utilise le couplage covalent entre une fonction -NH₂ portée par le saccharide et un groupement carbonyle présent sur la matrice permettant d'obtenir une liaison amide est décrit Il est également indiqué que la liaison amide peut être obtenue par réaction EDC/NHS (1-éthyl-3-(3-diméthyiaminopropyl)carbodiimide et N-hydroxysuccinimide) avec un groupe carboxylique sur la matrice qui est activée pour réagir avec le dérivé saccharidique. Aucun exemple plus précis n'est donné ;
- la demande de brevet EP 2556848 qui décrit un matériau de séparation comprenant un saccharide lié *via* un bras de liaison particulier à une matrice pour permettre de séparer des substances d'un liquide. Il est indiqué que la liaison oligosaccharide-matrice peut être obtenue en faisant réagir un composé sur une matrice porteuse de fonctions -NH₂, -N₃, -COOH, -CHO, NH₂-NH-, HC≡C- ou époxy (voir définition de F¹ page 4 ligne 7). La réaction de couplage détaillée au paragraphe [0069] précise que la liaison est réalisée entre un groupe carboxyle et une amine et qu'une des méthodes classiquement utilisées est la réaction d'un acide carboxylique avec un carbodiimide, pour faciliter son couplage avec une amine de manière à obtenir une liaison de type amide. Les supports détaillés dans les exemples incorporent exclusivement des fonctions époxy, amino et acide carboxylique.
- EP 0131546 A2 décrit un support pour la détection de certaines classes d'anticorps. Le support comprend une matrice solide dont la surface est modifiée avec un aldéhyde polyfonctionnel, préférentiellement du glutaraldéhyde, de façon à fournir une surface comprenant un grand nombre de fonctions aldéhyde libres. Sur cette surface sont ensuite fixés des allergènes et antigènes qui sont spécifiques des classes d'anticorps par simple incubation du support modifié par le glutaraldéhyde avec une solution d'antigènes ou anticorps. On soumet ensuite le support à un traitement au borohydrure de sodium.
- US 2004/242857 A1 décrit également un support pour la purification de liquides biologiques comprenant une matrice, une molécule de liaison ou espaceur entre ladite matrice et un ligand spécifique à la fixation par exemple d'anticorps anti-A ou anti-B. Ce ligand est un saccharide qui peut notamment être un di-, tri-, tetra- ou pentasaccharide.

Il apparaît donc que différentes solutions utilisant des oligosaccharides antigéniques d'un groupe sanguin greffé sur un support ont été proposées. La liaison oligosaccharide / support est le plus souvent réalisée sur un support porteur de fonctions -NH₂, époxy ou adde carboxylique.

Dans le cadre de l'invention, un nouveau support permettant notamment de favoriser la liaison des anticorps du type IgG et IgM sur des partenaires de liaison de nature oligosaccharidique, et en particulier la liaison des anticorps des groupes sanguins, est proposé.

La présente invention concerne un support pour la purification d'un liquide biologique comprenant une phase solide sur laquelle sont greffées, par liaison covalente, des molécules d'au moins un oligosaccharide capable de se lier à un ou plusieurs anticorps, ladite phase solide étant porteuse de fonctions aldéhyde -CHO libres, tel que défini à la revendication 1.

Dans le cadre de l'invention, les fonctions aldéhyde libres -CHO assurent une stabilisation des anticorps, consécutivement à leur reconnaissance immunologique par lesdites molécules d'oligosaccharide(s) greffées sur la phase solide. Les fonctions aldéhyde libres -CHO sont réparties sur la phase solide et viennent interagir avec les anticorps, initialement contenus dans le liquide biologique à purifier et qui viennent se lier par liaison d'immuno-affinité sur des molécules d'oligosaccharide(s) greffées sur la phase solide. En particulier, la stabilisation de la liaison oligosaccharide/anticorps est obtenue par la fixation, notamment par liaison covalente, avec l'un ou plusieurs des acides aminés suivants : asparagine, lysine, arginine, glutamine, tyrosine, tryptophane, ou cystéine, présent sur ledit anticorps, sur une fonction aldéhyde libre présente sur la phase solide. Les fonctions aldéhyde libres -CHO sont présentes directement sur la phase solide et nullement sur les oligosaccharides. La présente invention concerne également un procédé de purification d'un liquide biologique comprenant des anticorps, dans lequel le liquide biologique est mis en contact avec au moins un support selon l'invention, de manière à obtenir la capture d'anticorps présents dans ledit liquide biologique par liaison(s) avec au moins certaines des molécules d'oligosaccharide(s) greffées sur la phase solide. Aussi, de manière avantageuse, dans un tel procédé de purification, ledit au moins support est utilisé pour assurer, par l'intermédiaire d'au moins une partie des fonctions aldéhyde libres présentes sur la phase solide, une stabilisation des anticorps, et en particulier des IgG, qui viennent se lier à au moins certaines des molécules d'oligosaccharide(s) greffées sur ladite phase solide. Les fonctions aldéhyde libres -CHO peuvent interagir avec les anticorps et stabiliser leur liaison d'affinité au support de purification.

L'invention concerne également un procédé de préparation d'un support conforme à l'invention, dans lequel le greffage des molécules d'oligosaccharide(s) est réalisé sur une phase solide porteuse de fonctions aldéhyde -CHO libres, par réaction, sur une partie seulement des fonctions aldéhyde -CHO libres présentes sur la phase solide, d'un oligosaccharide porteur d'une fonction réactive apte à former une liaison covalente avec ladite fonction aldéhyde -CHO. La liaison covalente existant entre la phase solide et les molécules d'oligosaccharides greffées sur le support final résulte donc de cette réaction sur une partie des fonctions aldéhyde -CHO libres initialement présentes.

De manière préférée, les molécules d'oligosaccharide sont greffées sur la phase solide par réaction d'un oligosaccharide fonctionnalisé porteur d'une fonction amine du type -NH₂, -NH-NH₂ ou -O-NH₂, éventuellement sous forme protégée, et d'une fonction aldéhyde -CHO portée avant greffage par la phase solide.

Selon un mode de réalisation particulier, la phase solide, sur laquelle les molécules d'oligosaccharide(s) sont greffées est de la cellulose qui comporte des unités de formule :

-CH(CHO)-CH(CH₂OH)-O-CH(CHO)-O-.

La présente invention a également pour objet l'utilisation d'au moins un support selon l'invention pour la purification d'un liquide biologique contenant des anticorps à capturer, dans lequel ledit liquide biologique est mis en contact avec le au moins dit support pour assurer une stabilisation de la liaison des anticorps IgG, qui viennent se lier à au moins certaines des molécules d'oligosaccharide(s) greffées à la surface de la phase solide. En particulier, la stabilisation est assurée par l'intermédiaire d'au moins une partie des fonctions aldéhyde libres présentes sur la phase solide, qui, de préférence, interagissent avec une asparagine, lysine, arginine, glutamine, tryptophane, tyrosine, ou cystéine, présent sur lesdits anticorps pour former une liaison covalente.

### Phases solides utilisées

Dans le cadre de l'invention, différents types de phases solides pourront être utilisés. En particulier, on utilisera une phase solide insoluble dans une phase aqueuse. A titre d'exemple de phase solide, on peut citer les phases solides de nature organique, notamment du type polymérique, plastique, polysaccharidique ou protéique ... tels que le polystyrène, les polycarbonates, les polyacrylates, les polysulfones, les polyuréthanes, la cellulose, l'agarose, le dextrane, le chitosane, la polylysine, ou encore les phases solides de nature inorganique comme la silice ou l'alumine. La phase solide pourra se présenter sous la forme notamment de particules ou d'une matrice fibreuse ou d'un soluté pouvant être insolubilisé ou séparé de façon sélective.

Dans le cadre de l'invention, la phase solide a été sélectionnée pour permettre de greffer des oligosaccharides aminés, plus faciles à purifier. Dans l'art antérieur, le greffage d'oligosaccharides aminés était réalisé sur des phases solides porteuses de fonctions époxy ou acide carboxylique. Ces techniques antérieures par rapport à celles privilégiées dans le cadre de l'invention présentent des inconvénients : le greffage époxy amine est difficile à contrôler et celui sur un acide carboxylique fait intervenir des molécules EDC et/ou NHS qui complexifient la réaction de couplage.

Dans le cadre de l'invention, la phase solide est porteuse de fonctions aldéhyde libres : ces fonctions correspondent, en particulier, à des fonctions aldéhyde résiduelles restant sur la phase solide après greffage des molécules d'oligosaccharides, dont le greffage a été réalisé sur d'autres fonctions aldéhyde présentes initialement en surface de la phase solide.

Aussi, de manière avantageuse, dans les supports selon l'invention, les molécules d'oligosaccharide(s) sont greffées sur une phase solide porteuse de fonctions aldéhyde -CHO libres, par réaction, sur une partie seulement des fonctions aldéhyde -CHO libres présentes sur la phase solide, d'un oligosaccharide porteur d'une fonction réactive apte à former une liaison covalente avec ladite fonction aldéhyde -CHO. Cette réaction de greffage est menée de manière telle qu'au final, des fonctions aldéhyde libres subsistent sur la phase solide. La liaison covalente existant entre la phase solide et les molécules d'oligosaccharides greffées sur le support final correspond, de préférence, à une liaison résultant de la réaction d'une fonction amine du type -NH₂, -NH-NH₂ ou -O-NH₂, éventuellement sous forme protégée, portée avant greffage par l'oligosaccharide et d'une fonction aldéhyde -CHO portée avant greffage par la phase solide.

L'introduction des fonctions aldéhyde libres sur la phase solide est donc réalisée avant le greffage des molécules d'oligosaccharide et sera fonction de la nature de ladite phase solide. Les fonctions aldéhyde pourront, par exemple, être directement insérées lors de la polymérisation de la phase solide.

Il est également possible que la phase solide contienne un ou plusieurs types de fonctions réactives qui vont permettre l'introduction des fonctions aldéhyde libres. En particulier, la phase solide peut contenir un ou plusieurs types de fonctions réactives présentant un caractère électrophile, tels que les acides carboxyliques et dérivés activés (par exemple, les acides carboxyliques activés au NHS), les époxy, les isocyanates et les isothiocyanates. Ces fonctions pourront être dérivées en aldéhyde par réaction avec un réactif qui porte, d'une part, un thiol, un alcool, une fonction -NH₂, -NH-NH₂ ou -O-NH₂, et d'autre part, une fonction aldéhyde masquée comme un acétal. La fonction aldéhyde pourra être ensuite déprotégée par hydrolyse de l'acétal. Par exemple, on pourra greffer l'aminoacétaldéhyde diméthyl acétal par réaction de sa fonction amine sur une phase solide portant des fonctions époxy. L'aldéhyde sera ensuite libéré dans une solution acide.

De manière alternative, la phase solide peut contenir un ou plusieurs types de fonctions réactives présentant un caractère nucléophile, tels que les thiols, alcools, et les fonctions -NH₂, -NH-NH₂ et -O-NH₂. Une telle fonction réactive pourra être mise en réaction, soit avec une molécule présentant, d'une part, une fonction acide que l'on activera pour former une liaison amide avec la phase solide et d'autre part, une fonction aldéhyde masquée, soit avec une molécule avec deux fonctions aldéhyde telle que le glutaraldéhyde ou le diméthoxyéthanal, ce dernier devant alors subir une étape de déprotection.

De manière alternative, la phase solide peut contenir des fonctions hydroxyles, qui pourront également être déprotonnées par une base forte et mises en présence d'un réactif qui porte, d'une part, un halogène de type chlore, et d'autre part, une fonction aldéhyde masquée comme un acétal, tel que l'halogénoacétaldéhyde diméthylacétal. Il en résultera une fonction aldéhyde masquée liée *via* une liaison éther à la phase solide. Cette fonction sera alors déprotégée en aldéhyde. Ce même type de polymère hydroxylé peut être activé par du bromure de cyanogène qui pourra alors réagir également avec l'amine de l'aminoacétaldéhyde diméthyl acétal.

Dans le cas d'une phase solide minérale, telle que la silice, cette dernière pourra être fonctionnalisée par un trichlorosilane, triméthoxysilane ou triéthoxysilane comportant aussi une fonction réactive, aldéhydique ou autre pouvant être dérivée par la suite vers un aldéhyde, par exemple le (3-glycidyloxypropyl) triméthoxysilane.

Quelle que soit la nature de la phase solide (organique ou inorganique), il est également possible d'utiliser une phase solide porteuse de fonctions réactives en « click chemistry », pour l'introduction des fonctions aldéhyde libres. De telles fonctions réactives en « click chemistry » sont, par exemple, choisies parmi les fonctions thiol, maléimide, allyle, acrylate, azide, propargyle, ..., qui pourront être mises en réaction avec un réactif correspondant adapté pour introduire par « click chemistry » une fonction aldéhyde libre.

De manière avantageuse, le support selon l'invention comprendra une phase solide se présentant sous la forme de particules hydrophiles, du type cellulose. Un tel choix permet de concentrer les protéines du liquide biologique purifié grâce audit support.

Dans le cadre de l'invention, une phase solide en cellulose sera, de préférence, utilisée. La cellulose présente l'avantage d'être une phase solide économique qui, de plus du fait de son pouvoir absorbant, permettra de concentrer les liquides biologiques, tels que les produits issus du sang et en particulier les plasmas, qui seront purifiés avec le support selon l'invention, en retenant une partie de l'eau contenue dans lesdits liquides biologiques.

En effet, une phase solide telle que la cellulose présente l'avantage d'être facilement dispersée dans le milieu à traiter (sang ou produit issu du sang), d'offrir une grande surface spécifique accessible, d'avoir une bonne neutralité chimique et immunologique par rapport aux composants du sang humain. Son utilisation, de préférence sous forme de particules stables et aseptiques, n'entraîne pas de dilution des protéines plasmatiques. En effet, la concentration des protéines plasmatique est un effet favorable recherché.

Dans le cadre de l'invention, on pourra notamment utiliser une cellulose micronisée ou une cellulose microcristalline, en tant que phase solide.

La cellulose microcristalline est obtenue par désintégration mécanique contrôlée et hydrolyse acide des extrémités à partir de la cellulose micronisée obtenue elle-même par désintégration mécanique à partir de pulpage de matière végétale fibreuse cellulosique. Les extrémités fibreuses présentes sur la cellulose micronisée sont éliminées sur la cellulose microcristalline, plus compacte.

Dans le cas d'une phase solide choisie parmi les polysaccharides, et en particulier, dans le cas de la cellulose, les fonctions aldéhyde pourront être introduites sur la phase solide par oxydation périodique, oxydation au TEMPO (2,2,6,6-tetraméthylpipéridine-1-oxyl), oxydation de Swern, éthérification avec un aldéhyde masqué halogéné, dérivation de la cellulose en tosylate (-OTs) (sur lequel on fera réagir l'alcoolate du glycoladéhyde diméthyl acétal ou un -NH₂ par substitution), activation CNBr (suivie de la réaction avec l'aminoacétaldéhyde diméthyl acétal). Dans le cas d'acétals formés intermédiairement, ces derniers sont ensuite hydrolysés pour conduire à une fonction aldéhyde libre.

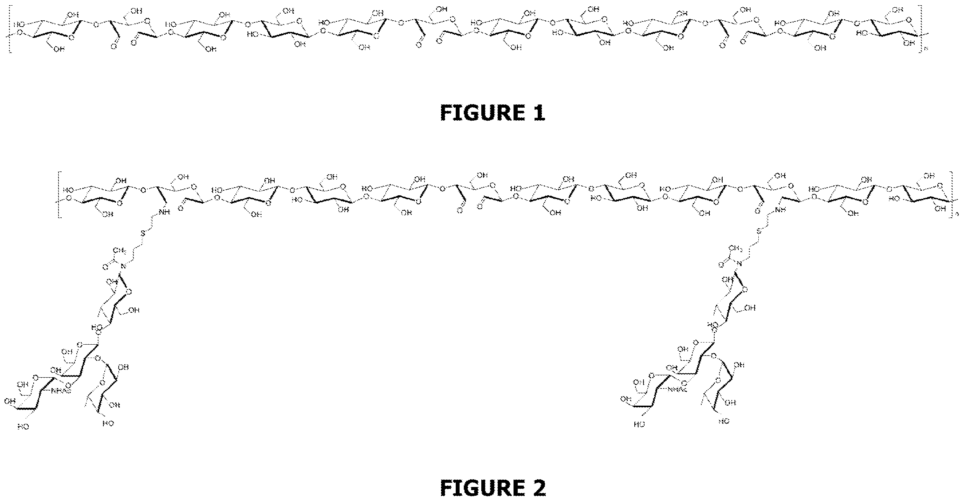

L'oxydation périodique plus particulièrement, entraîne la coupure d'une liaison C-C entre les deux carbones portant les deux alcools vicinaux d'une unité saccharidique et la création de deux fonctions aldéhyde. La **Figure 1** illustre la création de telles fonctions aldéhyde dans le cas de la cellulose. Deux fonctions aldéhyde à proximité l'une de l'autre sont donc créées. La création de fonctions aldéhyde de manière régulière et toutes les quatre unités glucosidiques présentée sur la **Figure 1** est donnée à titre purement illustratif. La création de fonctions aldéhyde pourra être régulière ou aléatoire et plus ou moins rapprochée. Aussi, de manière avantageuse, dans les supports selon l'invention, la phase solide est un polysaccharide et les fonctions aldéhyde libres résultent d'une oxydation périodique. De manière encore plus préférée, dans les supports selon l'invention, la phase solide est de la cellulose qui comporte des unités de formule :

-CH(CHO)-CH(CH₂OH)-O-CH(CHO)-O-.

En effet, certaines de ces unités peuvent subsister dans la phase solide porteuse des molécules d'oligosaccharide greffées.

Les conditions d'introduction des fonctions aldéhydes et, en particulier, de l'oxydation périodique, utilisées pourront être adaptées par l'homme du métier, en fonction de la densité de fonctions aldéhyde qu'il souhaite introduire sur la phase solide.

On pourra, notamment, se référer à "An introduction to the chemistry of carbohydrates", R.D. Guthrie & John Honeyman, Oxford University Press, 1968, page 108 à 112, pour les conditions à appliquer pour l'oxydation périodique de la cellulose, ou plus généralement des polysaccharides. En particulier, dans le cas de l'oxydation périodique, un periodate tel que le métaperiodate de sodium NaIO₄, sera utilisé. La réaction d'oxydation périodique sera le plus souvent réalisée sur la phase solide mise en suspension dans un solvant.

Par exemple, du métaperiodate de sodium NaIO₄ dissout dans une phase aqueuse est mis en réaction sur une phase solide du type polysaccharide telle que la cellulose en suspension. De manière avantageuse, la réaction est menée sous agitation de préférence dans l'obscurité, avec ou sans chauffage, et de préférence à une température appartenant à la gamme allant de 0 à 30°C, en milieu de préférence neutre ou acide. Le taux d'introduction de fonctions aldéhyde libres sur la phase solide du type polysaccharide telle que la cellulose dépend, en particulier, de la concentration en réactif utilisé, et en particulier en agent oxydant tel que le NaIO₄. Les conditions seront adaptées pour obtenir, de préférence, une fonction aldéhyde pour 100 à 4 monomères saccharidiques, préférentiellement une fonction aldéhyde pour 25 à 8 monomères saccharidiques.

Des phases solides déjà porteuses de fonctions aldéhyde libres, notamment sous la forme de groupes formyle sont également disponibles dans le commerce. On peut notamment citer les phases solides activées chromatographiques macroporeuses d'un polymère méthacrylique commercialisés par Tosoh Biosciences, notamment sous la référence Toyopearl AF-Formyl-650 ou des gels d'Agarose portant des fonctions aldéhydes. Une telle phase solide porteuse de fonctions aldéhyde libres est alors prête pour pouvoir être fonctionnalisée, par greffage covalent de molécules d'oligosaccharide(s), sur une partie des fonctions aldéhyde.

### Oligosaccharide et Greffage sur la phase solide

Il est possible de greffer sur la phase solide des molécules d'un seul oligosaccharide ou des molécules de plusieurs oligosaccharides différents.

Le ou les oligosaccharide(s) est (sont), de préférence, un trisaccharide, un tetrasaccharide, un pentasaccharide ou un hexasaccharide.

Le ou les oligosaccharides greffé(s) sur la phase solide est (sont) qualifié(s) d'antigénique(s), de par leur capacité à reconnaitre un ou plusieurs anticorps.

Dans le cadre de l'invention, seront, de préférence, greffées sur la phase solide des molécules d'au moins un oligosaccharide capable de se lier aux anticorps anti déterminant A, B ou H des groupes sanguins. Les anticorps anti déterminant A sont des anticorps réguliers présents chez les êtres humains de groupe sanguin B ou O ; les anticorps anti déterminant B sont des anticorps réguliers présents chez les êtres humains de groupe sanguin A ou O ; les anticorps anti déterminant H sont des anticorps présents chez certains êtres humains de groupe sanguin O. Les oligosaccharides présents sur les supports selon l'invention, qui sont capables de se lier aux anticorps anti déterminant des groupes sanguins, peuvent être de tout type : oligosaccharide antigénique du déterminant A, oligosaccharide antigénique du déterminant B notamment, par exemple sous leur forme naturelle hexasaccharidique ou réduite en taille (tri tétra ou penta-saccharidique).

Notamment, le ou les oligosaccharide(s) pourra(ont) être choisi(s) parmi les oligosaccharides capables de se lier aux anticorps anti déterminant B, présents chez les individus du groupe sanguin A ou O, tels que le tétrasaccharide de type 5 qui n'est pas présent dans le répertoire humain mais qui présente, de façon surprenante une excellente affinité pour les anticorps humains, l'hexasaccharide de type 1 et l'hexasaccharide de type 2 qui font partie du répertoire humain; parmi les oligosaccharides capables de se lier aux anticorps anti déterminant A, présents chez les individus du groupe sanguin B ou O, tels que le tétrasaccharide de type 5 qui n'est pas présent dans le répertoire humain mais qui présente, de façon surprenante une excellente affinité pour les anticorps humains, l'hexaose de type 1 et l'hexaose de type 2 qui font partie du répertoire humain.

De manière avantageuse, le support selon l'invention sera porteur de molécules d'un oligosaccharide capable de se lier aux anticorps anti déterminant B, présents chez les individus du groupe sanguin A ou O et/ou de molécules d'un oligosaccharide capable de se lier aux anticorps anti déterminant A, présents chez les individus du groupe sanguin B ou O. A titre d'exemples de tels oligosaccharides, on peut citer : GalNAcα1-3(Fucα1-2)Galβ1, Galα1-3(Fucα1-2)Galβ1, GalNAcα1-3(Fucα1-2)Galβ1-4Glc, Galα1-3(Fucα1-2)Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, GalNAcα1-3(Fucα1-2)Galβ1-4GlcNAcβ1-3Galβ1-4Glc, et Galα1-3(Fucα1-2)Galβ1-4GlcNAcβ1-3Galβ1-4Glc, Fucα1-2Galβ1-3GlcNAc, Fucα1-2Galβ1-4GlcNAc, Fucα1-2Galβ1-4Glc, Fucα1-2Galβ1-3GlcNAcβ1-3Gal, Fucα1-2Galβ1-4GlcNAcβ1-3Gal, Fucα1-2Galβ1-3GalNAcβ1-3Gal, Fucα1-2Galβ1-3GlcNAcβ1-3Galβ1-4Glc, Fucα1-2Galβ1-4GlcNAcβ1-3Galβ1-4Glc, les oligosaccharides GalNAcα1-3(Fucα1-2)Galβ1-4Glc et Galα1-3(Fucα1-2)Galβ1-4Glc étant préférés.

Il est possible de greffer sur la phase solide d'autres types d'oligosaccharides se liant à des anticorps présents dans des échantillons de sang ou de produits issus du sang, autres que ceux capables de se lier aux anticorps anti déterminant A, B, H des groupes sanguins. A titre d'exemples de tels oligosaccharides, on peut citer des oligosaccharides pouvant être impliqués dans des problèmes thérapeutiques :
- les xéno-antigènes, de différents types tels que le Xeno Lewis^{a} (notamment commercialisé par la société ELICITYL, sous la référence GLY076), Galα1-3Galβ1-3(Fucα1-4)GlcNAc, le Xeno Lewis^{x} (notamment commercialisé par la société ELICITYL, sous la référence GLY075), Galα1-3Galβ1-4(Fucα1-3)GlcNAc, le Xeno antigen type 2 (notamment commercialisé par la société ELICITYL, sous la référence GLY074-2), Galα1-3Galβ1-4GlcNAc et le Xeno antigen type 1 (notamment commercialisé par la société ELICITYL, sous la référence GLY074-1), Galα1-3Galβ1-3GlcNAc.
- les antigènes de Forssman, sous la forme d'un pentaose (notamment commercialisé par la société ELICITYL, sous la référence GLY132) GalNAcα1-3GalNAcβ1-3Galα1-4Galβ1-4Glc ou d'un triaose (notamment commercialisé par la société ELICITYL, sous la référence GLY133) GalNAcα1-3GalNAcβ1-3Gal.

Lors de la préparation des supports selon l'invention, les oligosaccharides devront être fonctionnalisés pour permettre leur greffage par liaison covalente sur la phase solide, par réaction sur une fonction aldéhyde présente en surface de la phase solide.

Une telle fonctionnalisation est, par exemple, la présence d'une fonction amine du type -NH₂, -NH-NH₂ ou -O-NH₂.

Pour cela, on pourra utiliser un oligosaccharide portant une fonction amine, amine protégée (sous forme de trifluoroacétate ou Boc, notamment). On pourra également introduire une telle fonction, en partant d'un oligosaccharide portant une fonction halogénée (par mise en œuvre d'une réaction de Gabriel par exemple), ou d'un oligosaccharide ayant réagi avec une hydrazine ou un de ses dérivés, ou d'un oligosaccharide activé pour la « click chemistry ». On peut également fonctionnaliser l'oligosaccharide par amination réductrice avec un réactif portant une fonction amine ou hydrazine et éventuellement une autre fonction qui pourra être dérivée par la suite.

L'introduction de telles fonctions par « click chemistry » pourra, par exemple, être réalisée en utilisant des oligosaccharides porteurs d'une fonction allyle et notamment d'une fonction -N(COCH₃)-CH₂-CH=CH₂ sur le carbone anomérique, position 1 du sucre en position réductrice obtenue par un procédé de fermentation bactérienne, notamment décrit dans la demande WO 01/04341, à laquelle on pourra se référer pour plus de détails. Cette fonction peut également être introduite sur l'oligosaccharide natif par réaction d'une amine, par exemple la propargylamine, sur l'aldéhyde de l'oligosaccharide et stabilisation de l'intermédiaire par acétylation pour former une fonction -N(COCH₃)-CH₂-CH=CH₂.

L'introduction d'une fonction amine sur l'oligosaccharide pourra se faire par réaction avec un réactif porteur d'une fonction amine à une extrémité et à l'autre extrémité, d'une fonction capable de réagir sur la fonction allyle portée par l'oligosaccharide. Une telle fonction sera, par exemple, une fonction thiol -SH, un diène conjugué (par réaction de Diels Alder). Il est également possible de réaliser sur la fonction allyle une ozonolyse suivie d'une amination réductrice avec l'ammoniac.

L'introduction d'une fonction amine pourra être également réalisée par une réaction de « click-chemistry », menée par réaction sur un oligosaccharide, porteur d'une fonction -C≡CH, et notamment propargyle. Cette fonction propargyle peut être introduite lors de la production de l'oligosaccharide par voie de fermentation suivant le procédé décrit dans la demande WO 01/04341, à laquelle on pourra se référer pour plus de détails. Cette fonction peut également être introduite sur l'oligosaccharide natif par réaction de l'amine, par exemple la propargylamine, sur l'aldéhyde de l'oligosaccharide et stabilisation de l'intermédiaire par acétylation pour former une fonction -N(COCH₃)-CH₂-C≡CH.

Dans ce cas, une réaction de « click-chemistry » sera menée avec un réactif porteur d'une part, d'une fonction azide -N₃, thiol -SH par exemple, et d'autre part, d'une fonction du type amine. La réaction entre une fonction -C≡CH et -N₃ conduit à la formation d'un groupe triazole. La réaction entre une fonction -C≡CH et un ou deux -SH conduit respectivement à la formation d'un ou de deux thioéther(s).

Différents bras de couplage pourront être présents dans ledit réactif (utilisé pour le greffage de l'oligosaccharide) entre la fonction destinée à établir le lien avec l'oligosaccharide (notamment -SH, -N₃) et la fonction (notamment amine du type -NH₂, -NH-NH₂ ou -O-NH₂) qui va être ultérieurement utilisée pour établir le greffage covalent de l'oligosaccharide sur la phase solide : notamment des bras de couplage du type polyéthylène glycol ou encore des chaînes alkylène du type -(CH₂)ₘ- avec m qui peut être 2, 3, 4 ou 5 notamment.

La quantité d'oligosaccharide(s) fonctionnalisé(s) mise en réaction sur la phase solide sera ajustée, de manière à obtenir un support avec le taux de greffage souhaité et un ratio fonctions aldéhyde libres présentes sur la phase solide après greffage / molécules d'oligosaccharide(s) greffées sur la phase solide, dans les gammes souhaitées. En particulier, une quantité suffisamment faible sera utilisée pour ne pas saturer les fonctions aldéhyde présentes sur la phase solide.

De manière avantageuse, dans les supports selon l'invention, le ratio nombre de molécules d'oligosaccharide(s) greffées sur la phase solide / nombre de fonctions aldéhyde libres présentes sur la phase solide appartient à la gamme allant de 1/400 à 1/1, de préférence à la gamme allant de 1/200 à 1/10 et/ou le support comporte de 1 à 100 mg d'oligosaccharide(s) /g de support, préférentiellement entre 2 et 40 mg d'oligosaccharide(s) /g de support.

On pourra se référer aux exemples pour la méthode de dosage du ou des oligosaccharide(s) greffé(s) et le calcul de la quantité d'oligosaccharide(s) greffé par gramme de support, ainsi que la méthode de dosage des fonctions aldéhyde libres et le calcul du ratio nombre de molécules d'oligosaccharide(s) greffées sur la phase solide / nombre de fonctions aldéhyde libres présentes sur la phase solide pouvant être utilisés.

Lors de la préparation des supports selon l'invention, le greffage d'oligosaccharide(s) fonctionnalisé(s) sera le plus souvent réalisé sur la phase solide mise en suspension dans un solvant. Le greffage est généralement réalisé en suspension dans un solvant aqueux par amination réductrice. Pour cela, la phase solide porteuse de fonctions aldéhyde libres est mise en suspension dans un tampon, notamment légèrement acide (par exemple de pH 6-7), en présence de l'oligosaccharide fonctionnalisé avec une fonction amine du type -NH₂, -NH-NH₂ ou -O-NH₂ et d'un réducteur qui ne réduit pas les fonctions aldéhyde libres ne réagissant pas avec les fonctions amine. La réaction entre les fonctions -NH₂ et une partie des fonctions aldéhyde libres forme un intermédiaire imine instable. Cet imine est réduit par le réducteur pour donner une liaison covalente -CH₂-NH-. En général, la réaction sera menée, à une température de l'ordre de 20 à 30°C, sous agitation pendant 2 à 10 heures. A titre d'exemple de réducteur sélectif, on peut citer le cyanoborohydrure de sodium. Par réducteur sélectif, on entend un agent réducteur venant stabiliser la fonction imine formée, sans réduire les fonctions aldéhyde libres n'ayant pas réagi avec les fonctions amine. Par exemple, un réducteur puissant comme le Borohydrure de sodium (NaBH₄) n'est pas approprié puisqu'il réduira aussi toutes les fonctions aldéhyde libres résiduelles présentes sur la phase solide.

De telles réactions de couplage sont connues pour les protéines et pourront être appliquées par analogie : on pourra notamment se référer à Immunology, 1971, 20, 1061. A Simple Method for Coupling Proteins to Insoluble Polysaccharides C. J. SANDERSON AND D. V. WILSON pour le greffage sur phase solide.

Au final, sur le support selon l'invention, la liaison covalente oligosaccharide/phase solide pourra être établie par l'intermédiaire de différents bras de liaison incluant notamment un groupe triazole ou une liaison -S- et/ou une chaîne polyéthylène glycol et/ou une ou plusieurs chaînes alkylène identiques ou différentes du type -(CH₂)ₘ- avec m qui peut être 2, 3, 4 ou 5 notamment. En particulier, un bras défini dans le sens oligosaccharide phase solide du type -N(COCH₃)-CH₂-CH₂-CH₂-S-(CH₂)ₚ- avec p qui peut être 2, 3, 4 ou 5, pourra être utilisé. Ce bras peut être obtenu à partir d'un oligosaccharide porteur d'une fonction allyle, celui-ci présentant une facilité de purification après fermentation et un bras relativement simple à synthétiser.

En particulier, dans les supports selon l'invention, les molécules d'oligosaccharide(s) sont greffées sur la phase solide par l'intermédiaire d'un enchaînement -NH-**L**- dans le sens phase solide oligosaccharide, et notamment -CH₂-NH-**L**, **L** étant un bras de liaison. Notamment, le bras de liaison **L** comporte un groupe triazole ou une liaison -S- et/ou une chaîne polyéthylène glycol et/ou une ou plusieurs chaînes alkylène identiques ou différentes du type -(CH₂)ₜ- avec t qui peut être 2, 3, 4 ou 5 par exemple. Notamment, le bras de liaison **L** correspondra à l'enchaînement : -CH₂-CH₂-S-CH₂-CH₂-CH₂-N(COCH₃)-, le groupe N(COCH₃) étant directement lié à une unité saccharidique de l'oligosaccharide, de préférence sur le carbone anomérique de ladite unité saccharidique.

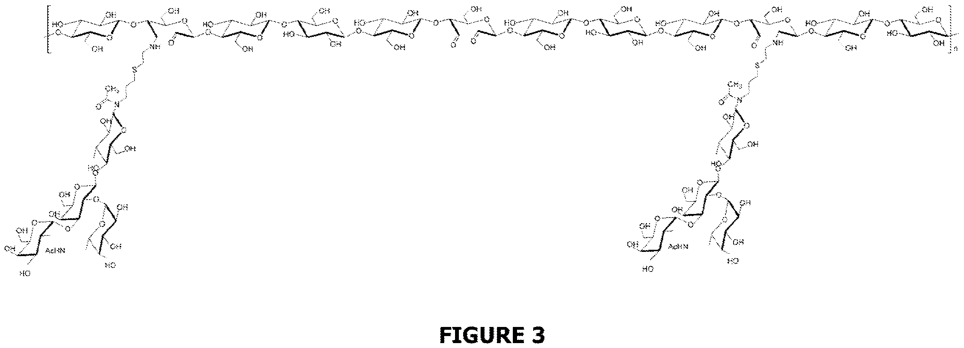

Après greffage des oligosaccharides, le support n'est soumis à aucun traitement qui pourrait affecter le caractère libre des fonctions aldéhyde résiduelles. En particulier, les fonctions aldéhyde résiduelles, présentes sur la phase solide après greffage des oligosaccharides, ne sont soumises à aucune réaction de réduction ou blocage, de manière à rester sous leur forme libre - CHO.On obtient, dans le cadre de l'invention, un support dont la phase solide est à la fois porteuse de fonctions aldéhyde libres résiduelles et de molécules d'oligosaccharide(s) greffées. Les **Figures 2** et **3** présentent une cellulose conforme à la **Figure 1** sur laquelle plusieurs molécules de deux exemples d'oligosaccharide ont été greffées. Là encore, l'alternance greffage sur une fonction aldéhyde, absence de greffage et greffage sur l'autre fonction aldéhyde présentée sur les **Figures 2** et **3** est donnée à titre purement illustratif. Le greffage pourra être régulier ou aléatoire et plus ou moins rapproché. Par contre, dans les supports selon l'invention dont la phase solide présente une structure saccharidique telle que la cellulose dans laquelle les fonctions aldéhyde ont été obtenues par oxydation périodique, il y aura présence de molécules d'oligosaccharide(s) greffées sur une unité saccharidique oxydée encore porteuse d'une fonction aldéhyde libre. En effet, le greffage de deux oligosaccharides sur deux fonctions aldéhyde libres d'une même unité saccharidique ouverte de la phase solide est défavorable. Aussi, l'aldéhyde vicinal d'une unité saccharidique ouverte porteuse d'un oligosaccharide est très majoritairement libre. Une telle configuration vient stabiliser la liaison oligosaccharide/anticorps.

Selon un mode de réalisation préféré, dans les supports selon l'invention, la phase solide est de la cellulose greffée qui comporte des unités de formule :

-CH(CHO)-CH(CH₂OH)-O-CH(CH₂-NH-**L**-oligo)-O-

et/ou des unités de formule :

-CH(CH₂-NH-**L**-oligo)-CH(CH₂OH)-O-CH(CHO)-O-

dans lesquelles **L** est un bras de liaison et **oligo** est un oligosaccharide capable de se lier à un ou plusieurs anticorps, de préférence un oligosaccharide capable de se lier aux anticorps anti déterminant A, B ou H des groupes sanguins.

Néanmoins, les exemples de réalisation réalisés sur des supports du type polymère méthacrylique présentant des fonctions aldéhyde libres introduites de manière plus aléatoire montre qu'une telle configuration, bien que favorable, n'est pas strictement nécessaire pour obtenir la stabilisation de la liaison oligosaccharides/anticorps, la présence de fonction aldéhyde libres réparties sur le support étant suffisante.

Dans le cadre de l'invention, contrairement à ce qui est classiquement fait pour le couplage notamment de protéines (A Simple Method for Coupling Proteins to Insoluble Polysaccharides C. J. SANDERSON AND D. V. WILSON, Immunology, 1971, 20, 1061), on ne procède pas à une passivation, notamment par réduction, des fonctions aldéhyde libres restantes. En effet, dans le cadre de l'invention, comme cela ressortira dans les exemples, il a été mis en évidence que la présence de fonctions aldéhyde libres restantes permettait de stabiliser la liaison d'affinité créée entre les oligosaccharides et les anticorps auxquels ils se lient.

### Procédé de purification

Les supports, selon l'invention, pourront être utilisés dans toute technique d'immuno-affinité, par exemple par chromatographie ou par adsorption sur une suspension du support (communément appelé mode batch). Les supports selon l'invention permettent de purifier tout type de liquide biologique contenant des anticorps pour lesquels le ou les oligosaccharides présents sur le support présentent une affinité. Le support selon l'invention va permettre d'extraire les anticorps qui vont venir se lier à des molécules d'oligosaccharide(s) greffées sur la phase solide du support selon l'invention.

Par « liquide biologique », on entend tout liquide issu d'au moins un prélèvement clinique réalisé chez un être humain ou un animal. Un tel liquide biologique est, par exemple, du sang total, du plasma, du sérum, de l'urine, du liquide céphalo-rachidien, un liquide issu de selles, de prélèvements de nez, de gorge, de peau, de plaie, d'organes, de tissus ou de cellules isolées etc. Le liquide biologique pourra correspondre à un liquide biologique issu d'un unique individu ou à un mélange de différents liquides, de préférence de même nature (pool de plasmas, par exemple) provenant de différents individus, et notamment de différents êtres humains.

Par « produit issu du sang », on entend notamment un sérum, un plasma sanguin (c'est-à-dire la partie liquide du sang constituée d'eau, de sels minéraux, de molécules organiques (protides, lipides, glucides) dans laquelle se trouvent en suspension hématies, leucocytes, et plaquettes) ou une fraction plasmatique ou sérique, provenant d'un seul individu ou d'individus différents.

L'invention est particulièrement adaptée à la purification du sang total ou d'un produit issu du sang, en particulier en choisissant une phase solide sur laquelle sont greffées des molécules d'oligosaccharide(s) venant capturer les anticorps des groupes sanguins présents.

On utilisera, de préférence, un support selon l'invention porteur de molécules d'oligosaccharide(s) capable(s) de se lier aux anticorps anti déterminant A pour purifier des sangs de groupe B ou produits issus du sang de groupe B et un support selon l'invention porteur de molécules d'oligosaccharide(s) capable(s) de se lier aux anticorps anti déterminant B pour purifier des sangs de groupe A ou produits issus du sang de groupe A. Pour purifier des pools de plasmas ou des plasmas de groupe O, on utilisera soit deux types de supports selon l'invention : l'un porteur de molécules d'oligosaccharide(s) capable(s) de se lier aux anticorps anti déterminant A pour éliminer les anticorps anti-A et l'autre porteur de molécules d'oligosaccharide(s) capable(s) de se lier aux anticorps anti déterminant B pour éliminer les anticorps anti-B ; soit un support selon l'invention porteur à la fois de molécules d'oligosaccharide(s) capable(s) de se lier aux anticorps anti déterminant A et de molécules d'oligosaccharide(s) capable(s) de se lier aux anticorps anti déterminant B.

Une étape préalable d'équilibrage, préalable à la mise en contact du support selon l'invention avec le produit issu du sang à traiter, pourra être utilisée, notamment une étape d'équilibrage dans un tampon de type PBS ou un tampon salin contenant un anticoagulant (tel que ACD, acide citrate dextrose ou CPD citrate phosphate dextrose), notamment à un pH appartenant à la gamme allant de 5,5 à 9,5 et notamment dans la gamme allant de 6,4 à 7,8.

Dans le cas d'une purification par adsorption sur une suspension du support selon l'invention, la séparation du liquide biologique traité et du support sera réalisée, par exemple, par une séparation du type sédimentation ou centrifugation.

Selon un mode de réalisation particulier applicable à la purification de sang total, le support peut avantageusement être intégré dans un circuit de plasmaphérèse comportant une poche de prélèvement de sang reliée à un filtre de déleucocytation lui-même relié à une poche de transfert, reposant sur un agitateur, la poche de prélèvement contenant le support en quantité suffisante pour permettre de réaliser en partie simultanément la déleucocytation du sang total et la capture des anticorps, et en particulier des anticorps anti déterminant A, B et/ou H. Dans ce mode de réalisation, le sang ainsi déleucocyté et épuré en anticorps pourra être utilisé tel quel ou être séparé en fraction plasmatique et érythrocytaire.

Les supports de l'invention devront, de préférence, être utilisés comme consommables pour éviter tout risque de contamination croisée entre la purification de deux liquides biologiques successifs. Le coût de production devra, bien entendu, être compatible avec ce choix d'utilisation.

La présente invention propose, en particulier, un support permettant d'obtenir des plasmas sanguins ou des préparations plasmatiques permettant de réduire la concentration en anticorps des groupes sanguins présents, en dessous des seuils acceptables, que les anticorps soient du type IgG ou IgM. En ajustant la nature des oligosaccharides présents et en utilisant de manière combinée à la fois au moins un oligosaccharide capable de se lier aux anticorps anti déterminant A et au moins un oligosaccharide capable de se lier aux anticorps anti déterminant B, il est possible d'obtenir des plasmas sanguins ou des préparations plasmatiques dits universels car ne présentant aucun risque d'administration à des humains quel que soit leur groupe sanguin A, B ou O.

La présence de fonctions aldéhyde libres sur la phase solide permet de stabiliser la liaison oligosaccharide/anticorps, en particulier dans le cas des anticorps du type IgG et IgM, cette stabilisation pouvant être plus marquée dans le cas des IgG. Cette stabilisation peut intervenir par la fixation, notamment par liaison covalente, entre une asparagine, lysine, arginine ou glutamine présente sur l'anticorps et une fonction aldéhyde libre présente sur la phase solide du support selon l'invention. De même, cette stabilisation peut être réalisée par l'intermédiaire d'un acide aminé présentant un pKa alcalin, comme la tyrosine, le tryptophane, ou la cystéine, présent sur l'anticorps lié à un oligosaccharide et une fonction aldéhyde libre présente sur la phase solide du support selon l'invention. Dans le cas de l'asparagine, le micro-environnement créé par la fixation de l'anticorps sur l'oligosaccharide favorise la N-glycation non enzymatique d'une fonction aldéhyde libre située sur le support à proximité de l'oligosaccharide greffé (et en particulier l'aldéhyde vicinal dans le cas d'un support du type cellulose comportant des unités -CH(CHO)-CH(CH₂OH)-O-CH(CH₂-NH-L-oligo)-O-) sur une asparagine de l'anticorps. La stabilisation peut également se faire par formation d'un conjugué du type base de Schiff, intervenant avec un acide aminé de l'anticorps fixé comprenant une fonction amine libre, qui formera une liaison covalente de type -C=N- qui pourra être réduite dans le cas d'un liquide biologique qui présenterait au site de fixation de l'anticorps un milieu réducteur, la liaison formée serait encore plus stable.

Les exemples ci-après permettent d'illustrer l'invention, mais n'ont aucun caractère limitatif.

### Appareillages et Conditions utilisés pour les Caractérisations

### Mesure d'absorbance

La lecture de l'absorbance est réalisée sur un spectrophotomètre UNICO Spectrophotometer 1200P. L'échantillon est transféré dans une cuve spécifique en PMMA.

### HPAEC-PAD

Le profil en monosaccharides est déterminé par HPAEC sur une chaine chromatographique ICS2500 Dionex. La séparation est réalisée sur une colonne Dionex CarboPac PA1. L'élution est réalisée avec un gradient de soude, d'acétate de sodium et d'eau désionisée et la colonne est thermostatée à 17°C. Les conditions d'élution sont détaillées dans le **Tableau 1** ci-après :

**Tableau 1**

| **Temps (mn)** | **NaOH 200 mM** | **eau désionisée** | **NaOH 200 mM Acétate 1M** |
|---|---|---|---|
| 0 | 9,0% | 91,0% | 0,0% |
| 20 | 9,0% | 91,0% | 0,0% |
| 20,1 | 7,0% | 91,0% | 2,0% |
| 35 | 0,0% | 50,0% | 50,0% |
| 35,1 | 50,0% | 0,0% | 50,0% |
| 50 | 50,0% | 0,0% | 50,0% |
| 50,1 | 9,0% | 91,0% | 0,0% |
| 65 | 9,0% | 91,0% | 0,0% |

La détection est assurée par ampèrométrie pulsée sur une électrode d'or. Chaque monosaccharide est calibré par un étalonnage externe.

### MALDI TOF

Les spectres de masse sont obtenus sur un spectromètre de masse Maldi-ToF-ToF Speed (Bruker Daltonics). L'échantillon est préparé en solution à 2.10⁻⁴ M dans l'eau. On dépose sur une plaque 0,5 µL de la solution d'échantillon préparée avec 0,5 µl de DHB (acide 2,5-dihydroxybenzoïque) à 50 mg/mL. On laisse sécher à température ambiante ou sous un flux d'air à température ambiante. Après séchage du dépôt, la mesure est réalisée avec le spectromètre de masse. 10 ensembles de 1000 tirs chacun sont réalisés à différents endroits du dépôt. L'ensemble des 10 000 acquisitions constitue le spectre final. L'échelle des masses est ajustée par le passage d'une solution de calibration « Brucker peptide calibration standard 2 ».

### RMN du proton

L'acquisition des spectres RMN du proton est réalisée sur un spectromètre de résonnance magnétique Avance III 400 MHz Bruker. L'échantillon, sous forme lyophilisé, est préparé dans une solution de D₂O, à raison d'environ exactement 5 à 10 mg dans 700 µL de D₂O comportant un étalon interne, le succinate de sodium à 1,77 g/L. L'acquisition est réalisée dans un tube RMN, à 80°C avec un temps de relaxation d1 de 20 secondes et avec 16 scans. Les données brutes d'acquisition sont traitées avec le logiciel Topspin NMR de Brucker.

### Méthode de dosage des molécules d'oligosaccharide(s) greffées et calcul de la quantité d'oligosaccharide(s) greffée par gramme de support

Le taux de greffage en oligosaccharide(s) est mesuré par analyse des monosaccharides constitutifs après hydrolyse, par exemple à l'acide trifluoroacétique (TFA), du support selon l'invention conduisant à l'hydrolyse totale de l'oligosaccharide. Pour cela, on peut utiliser le protocole type suivant : 20 mg de support selon l'invention sont hydrolysés avec 500 µL de TFA 2N pendant 4h à 99°C. Le mélange est ensuite neutralisé avec 53 µL de NaOH 19N et ajusté avec à 1mL avec de l'eau désionisée. Un standard de l'oligosaccharide dans sa forme mise en œuvre pour le greffage, ou de préférence dans sa forme non fonctionnalisée, est traité dans des conditions identiques. Après filtration et dilution au quart, le profil monosaccharidique est déterminé par HPAEC-PAD. La comparaison des résultats obtenus pour le support selon l'invention et le standard, sur le dosage d'unités saccharidiques spécifiques des oligosaccharides non présents dans la phase solide utilisée, notamment du fucose et du galactose dans le cas des oligosaccharides utilisés dans les exemples qui sont non présents sur la cellulose utilisée dans certains des exemples en tant que phase solide, conduit soit directement à la quantité greffée d'oligosaccharide dans le cas où le standard est l'oligosaccharide dans sa forme réductrice non fonctionnalisée, soit à la quantité greffée d'oligosaccharide + bras de liaison dans le cas où le standard est l'oligosaccharide dans sa forme mise en oeuvre pour le greffage. Dans ce cas, la quantité d'oligosaccharide greffée peut directement être obtenue après déduction de la masse du bras de liaison.

### Méthode de dosage des fonctions aldéhyde libres et calcul du ratio nombre de molécules d'oligosaccharide(s) greffées sur la phase solide / nombre de fonctions aldéhyde libres présentes sur la phase solide

Le nombre de fonctions aldéhyde présentes sur la phase solide peut être mesuré par dosage colorimétrique avec de l'acide 3,5-dinitrosalycilique (DNS) qui va être réduit en acide 3-amino-5-nitrosalicylique (composé rouge) par les fonctions aldéhyde libres présentes, avec un étalonnage externe réalisé par exemple avec du glucose. Un protocole type est donné à l'exemple 1. On obtient ainsi le taux de fonctions aldéhydes par quantité de masse de support, et plus particulièrement, pour la cellulose, ce taux peut être exprimé par unité polymérique de glucose en considérant une masse molaire de 162 g/mol par unité glucose dans le polymère de cellulose.

Les dosages des oligosaccharides greffés par unité de masse de support ou par monomère de glucose de support pour la cellulose, rapportés au dosage des fonctions aldéhydes par unité de masse de support ou par monomère de glucose de support pour la cellulose, permettent de déterminer le ratio nombre de molécules d'oligosaccharide(s) greffées sur la phase solide / nombre de fonctions aldéhyde libres présentes sur la phase solide.

Les oligosaccharides qui vont être greffés sont les suivants :
La dénomination GrA signifie que l'oligosaccharide est capable de se lier aux anticorps anti déterminant A, présents chez les individus du groupe sanguin B ou O et GrB signifie que l'oligosaccharide est capable de se lier aux anticorps anti déterminant B, présents chez les individus du groupe sanguin A ou O.
6GrA1 : GalNAcα1-3(Fucα1-2)Galβ1-3GlcNAcβ1-3Galβ1-4Glc : 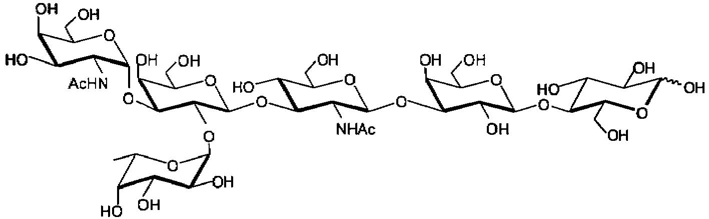
6GrB1 : Galα1-3(Fucα1-2)Galβ1-3GlcNAcβ1-3Galβ1-4Glc 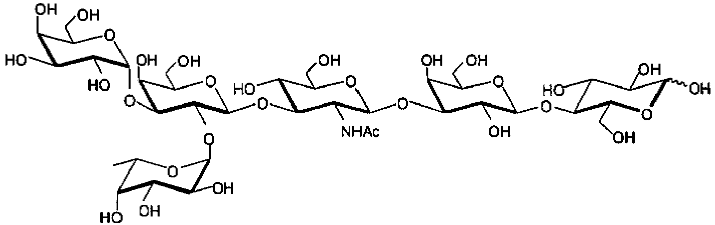
4GrA5 : GalNAcα1-3(Fucα1-2)Galβ1-4Glc 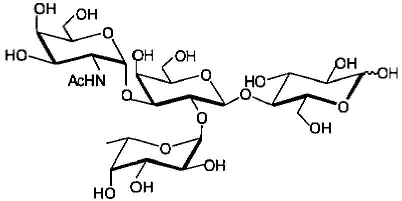
4GrB5 : Galα1-3(Fucα1-2)Galβ1-4Glc 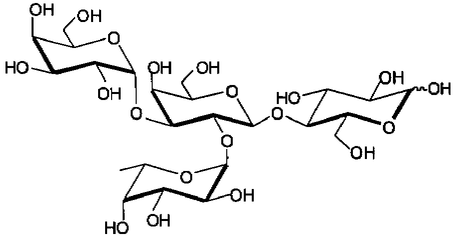
3GrA : GalNAcα1-3(Fucα1-2)Gal

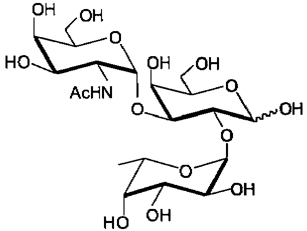

Les exemples, ci-après, permettent d'illustrer l'invention, mais n'ont aucun caractère limitatif.

### I. PREPARATION DES SUPPORTS

### Exemple 1 : Activation de cellulose micronisée et greffage de l'oligosaccharide 6GrA1-NAc Propargyl préalablement activé avec une fonction amine

De la cellulose micronisée, Vitacel 600-30 produite par Rettenmaier, est activée par la création de fonctions aldéhyde, obtenues par un traitement au métaperiodate NaIO₄. Pour cela, on mélange 500 g de cellulose micronisée avec 100 g de NaIO₄ et 5L d'eau. Le mélange est maintenu à température ambiante une nuit, sous agitation, puis, le support cellulosique obtenu est rincé à l'eau, puis à l'éthanol et séché à l'étuve à une température d'environ 50°C.

Le nombre de fonctions aldéhyde ainsi créées sur le support cellulosique est mesuré par dosage colorimétrique des fonctions réductrices. Pour cela, 5 mg de la cellulose activée obtenue sont solubilisés dans 500 µL d'eau désionisée. On ajoute 500 µL de réactif au DNS à 10g/l (1,6 g de NaOH dans 15 mL d'eau désionisée, 1 g d'acide 3,5 dinitrosalicylique (DNS) dans 20mL d'eau désionisée, 30g de tartrate double de sodium et de potassium, ajusté à 100mL avec de l'eau désionisée). Après 5 minutes à 95°C, l'échantillon obtenu est refroidi rapidement sous l'eau froide. On ajoute alors 5mL d'eau désionisée. Après un repos de 20mn, on effectue une lecture de l'absorbance à 546 nm. Le résultat obtenu est comparé à une courbe d'étalonnage externe réalisée dans les mêmes conditions avec du Glucose (comportant un aldéhyde par molécule). On en déduit que la cellulose activée obtenue comporte 1 fonction aldéhyde pour 14 glucoses. En réalisant 10 fois le dosage, il apparaît que la précision de la mesure est de 3%. Le pourcentage de précision est estimé par le rapport de l'écart type sur la moyenne des mesures. L'écart type étant défini par la racine carrée de la somme des écarts à la moyenne au carré divisé par le nombre de mesures moins une.

Une étape d'activation préalable de l'oligosaccharide 6GrA1-NAc-Propargyl est nécessaire pour permettre son greffage sur la cellulose activée par des fonctions aldéhyde obtenue. La GlycoBrick 6GrA1-NAc-Propargyl fournie par Elicityl (OligoTech^{®} Ref GLY037-1-NPR) est activée par ajout d'une molécule espaceur amine comportant une fonction réactive azide, une chaîne de 3 PEG et une fonction amine, le 11-Azido-3,6,9-trioxaundecan-1-amine (TCI Chemical référence A2363). La GlycoBrick 6GrA1-NAc-Propargyl est mise en solution dans un mélange Méthanol/Eau (50/50) à 10 g/L en présence de 1,2 eq. d'ascorbate de sodium, 0,4 eq. de CuSO₄ et 1 équivalent d'azido espaceur amine, le 11-azido-3,6,9-trioxaundecan-1-amine. Après environ 2 heures sous agitation, le produit de la réaction, 6AGrA1 aminé est purifié par passage sur une résine échangeuse de cations. Pour cela, la solution est mise en contact avec de la résine Dowex^{®} 50. Après élimination de la solution initiale et rinçage à l'eau, la molécule est désorbée avec une solution d'ammoniaque à 2%. Après évaporation et lyophilisation, le 6GrA1 aminé de formule : 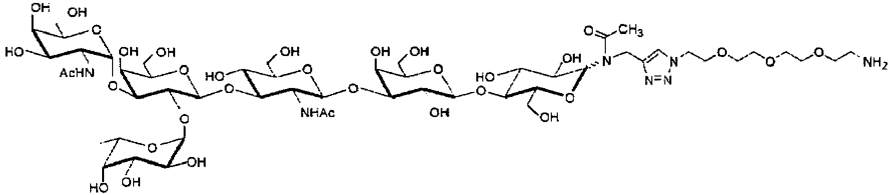 est prêt pour le greffage.

Le greffage du 6GrA1 aminé sur la cellulose activée obtenue précédemment est réalisé de la manière suivante : 15 g de cellulose activée, 6000 µL d'une solution de 6GrA1 aminé dans l'eau à 50 g/L, 2400 µL de tampon phosphate 0,5M pH3 et 36,600 mL d'eau sont mélangés pendant environ 16 heures à température ambiante. 15 mL d'une solution de NaCNBH₃ à 100g/L dans l'eau sont alors ajoutés. On laisse réagir 8 h sous agitation. Le support greffé est ensuite abondamment rincé à l'eau, puis à l'éthanol pur et est ensuite séché à une température de l'ordre de 50°C.

Le taux de greffage de 6GrA1 aminé est évalué par analyse des monosaccharides constitutifs après hydrolyse totale de l'oligosaccharide. Pour cela, 20 mg de la cellulose fonctionnalisée obtenue sont hydrolysées avec 500 µL de TFA 2N pendant 4h à 99°C. Le mélange est ensuite neutralisé avec 53 µL de NaOH 19N et ajusté à 1mL avec de l'eau désionisée. Un standard de 6GrA1 aminé est traité dans des conditions identiques. Après filtration et dilution au quart, le profil monosaccharidique est déterminé par HPAEC-PAD. La comparaison des résultats sur le dosage du fucose et du galactose spécifique des oligosaccharides, mais non présents sur la cellulose, entre la cellulose greffée et le standard, conduit à une quantité greffée de 6GrA1 aminé de 12,0 mg/g de support final, correspondant à une quantité de 6GrA1 (après soustraction de la masse correspondant au bras de liaison) greffé de 9,4 mg/g de support final. En réalisant 10 fois le dosage, il apparaît que la précision de la mesure est de 3,4%. Le pourcentage de précision est estimé par le rapport de l'écart type sur la moyenne des mesures. L'écart type étant défini par la racine carrée de la somme des écarts à la moyenne au carré divisé par le nombre de mesures moins une. Ceci correspond à 688 moles d'unités glucose de la cellulose pour 1 mole d'oligosaccharide greffé. Le taux d'aldéhydes résiduels après greffage est déterminé selon la méthode décrite à l'exemple 1, il est ici de 1 pour 18 (un aldéhyde pour 18 unités glucose, monomère de la cellulose).

Le ratio nombre de molécules d'oligosaccharide(s) greffées sur la phase solide/nombre de fonctions aldéhyde libres présentes sur la phase solide est égal à 1/38.

### Exemple 2 : Greffage de l'oligosaccharide 6GrB1-NAc Propargyl préalablement activé avec une fonction amine, sur de la cellulose micronisée préalablement activée

On utilise la cellulose micronisée préalablement activée, produite à l'exemple 1, comprenant 1 fonction aldéhyde pour 14 glucoses.

Une étape d'activation préalable de l'oligosaccharide 6GrB1-NAc-Propargyl (GlycoBrick 6GrB1-NAc-Propargyl fournie par Elicityl, OligoTech^{®} Ref GLY040-1-NPR) est réalisée dans les mêmes conditions que celles utilisées à l'exemple 1 pour l'oligosaccharide 6GrA1-NAc-Propargyl et conduit à l'oligosaccharide 6GrB1 aminé de formule suivante :

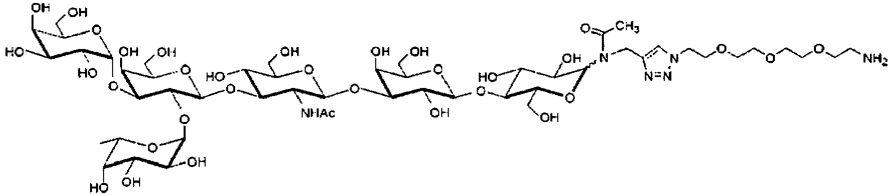

Le greffage du 6GrB1 aminé sur la cellulose activée est réalisé comme à l'exemple 1.

Le taux de greffage de 6GrB1 aminé est évalué selon la méthode détaillée à l'exemple 1 et permet de déterminer une quantité greffée de 6GrB1 aminé de 12,9 mg/g de support final, correspondant à une quantité de 6GrB1 (après soustraction de la masse correspondant au bras de liaison) greffé de 10,0 mg/g de support final. Ceci correspond à 620 moles d'unités glucose de la cellulose pour 1 mole d'oligosaccharide greffé. Le taux d'aldéhydes résiduels après greffage est déterminé selon la méthode décrite à l'exemple 1, il est ici de 1 pour 19 unités glucose.

Le ratio nombre de molécules d'oligosaccharide(s) greffées sur la phase solide/nombre de fonctions aldéhyde libres présentes sur la phase solide est égal à 1/33.

### Exemple 3 : Préparation de 4GrA5-S-(CH₂)₂-NH₂ et son greffage, sur de la cellulose micronisée préalablement activée

### 1) Préparation du précurseur de fermentation Lactose-NAc-Allyle

On fait réagir 25 g de lactose déshydraté (Meggle DuraLac^{®} H) avec 50 mL d'allyle amine. Après 12 heures de réaction à 43°C sous agitation avec un barreau magnétique, le mélange est séché à l'évaporateur rotatif sous pression réduite à 40-45°C. On co-évapore ensuite à trois reprises, avec à chaque fois 100mL de méthanol et 100 mL de toluène. On reprend l'ensemble dans 250 mL de méthanol auxquels on ajoute 200 mL d'anhydride acétique. On laisse réagir sous agitation et à température ambiante pendant 12 heures. Le mélange est ensuite séché à l'évaporateur rotatif sous pression réduite à 40-45°C. On co-évapore ensuite à trois reprises avec à chaque fois 100mL de méthanol et 100 mL de toluène. Le mélange réactionnel est repris avec 200 mL d'eau désionisée, puis lyophilisé. Le lyophilisat est repris dans 350 mL d'éthanol. On ajuste le pH à 9 avec du méthylate de sodium. On laisse réagir trois heures environ sous agitation et à température ambiante. On ajoute ensuite environ 200 mL d'eau désionisée et on neutralise l'ensemble avec de l'acide chlorhydrique.

### 2) Production du 4GrA5-Allyle par fermentation

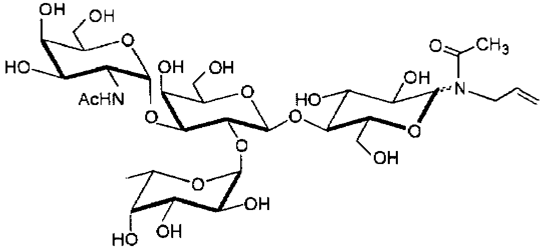

La production de 4GrA5-Allyle est réalisée en fournissant à une souche d'E. Coli modifiée du lactose Allyle préparé précédemment (procédé décrit dans le brevet FR2796082). La souche productrice de 4GrA5 est référencée SD142 par Elicityl (Randriantsoa, m. (2008). Synthèse Microbiologique des antigènes glucidiques des groupes sanguins. Grenoble : thèse de l'université Joseph Fourrier). Un fermenteur contenant 3,7L de milieu de culture (glycérol (2,4 g/L), NH₄H₂PO₄ (7 g/L), KH₂PO₄ (7 g/L), solution de traces minérales (7,5 mL/L), acide citrique (0,6 g/L), KOH (2,5 g/L), NaOH (1 g/L), MgSO₄ 7H₂O (1 g/L), Thiamine (4,5 mg/L)+ Glucose 7,5 g/L) est ensemencé par 80 mL d'une pré-culture de la journée (à une Densité Optique de 2). L'oxygène dissout est maintenu à 40%, le pH régulé à 6,8 par addition de NH₃ et la température maintenue à 34°C. La culture comporte trois phases. La première phase dure jusqu'à la consommation totale du glucose, puis du glycérol qui ont été mis au départ dans le milieu de culture. Au début de la seconde phase, les gènes codant pour les différents nucléotides synthétases et glycosyltransférases sont induits par ajout d'IPTG (isopropyl β-D-1-thiogalactopyranoside), les substrats sont rajoutés au milieu de culture, et l'alimentation à haut débit (40 mL/h) est lancée pour 5h. Durant la troisième phase, le débit de l'alimentation est réduit à 25 mL/h. La culture est arrêtée 48h après l'induction.

### 3) Purification du 4GrA5-Allyle produit par fermentation

Le milieu de culture obtenu précédemment est centrifugé pendant 20 mn à 8000 rpm. Le culot bactérien est récupéré, puis remis en suspension dans 2L d'eau désionisée équivalent au volume de culture. Le culot bactérien en suspension est porté à ébullition pendant 70 mn pour perméabiliser les bactéries. Les débris cellulaires sont éliminés par centrifugation pendant 20 mn à 8000 rpm. Le surnageant est acidifié à un pH 3 avec de l'acide chlorhydrique. Le précipité est éliminé par centrifugation pendant 20 mn à 8000 rpm. On neutralise le surnageant à la soude. On ajoute de la résine Dowex^{®} optipore SD2 et on laisse pendant 12 heures sous agitation. Après élimination de la résine, la solution est concentrée à environ 500 mL à l'évaporateur rotatif sous pression réduite. La solution est ensuite purifiée en chromatographie HPLC à polarité inverse de phase sur un support C18 lichrosphère 12µm de la société Merck. L'élution est réalisée avec un gradient d'éthanol entre 0 et 9% en volume. Le 4GrA5-Allyle est élué, concentré à l'évaporateur rotatif sous pression réduite et lyophilisée. On obtient par cette méthode de purification 6,8 g de 4GrA5-Allyle. L'identité et la structure de l'oligosaccharide sont validées en spectrométrie de masse MALDI TOF et RMN du proton. La spectrométrie de masse MALDI TOF permet de déterminer la masse attendue de 772 g/mol, c'est-à-dire sous sa forme complexée au sodium 795 g/mol. Le profil 1H RMN était conforme à la structure. La 1H RMN quantitative avec étalonnage interne permet d'estimer la pureté à 95% +/- 1,9%.

### 4) Activation du 4GrA5-Allyle par introduction d'une fonction amine

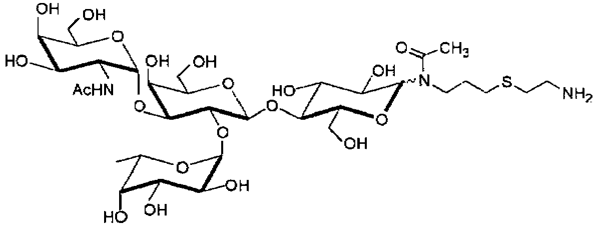

Le 4GrA5-Allyle obtenu précédemment est aminé par réaction de la fonction allyle avec la fonction thiol de la cystéamine. Pour cela, on mélange 500 mg de 4GrA5-Allyle produit précédemment avec 656 mg de cystéamine et 16,3 mg de d'acide 4-cyanovalérique dans 5 mL d'eau désionisée. La solution est maintenue deux heures sous agitation à 80°C. L'oligosaccharide obtenu, le 4GrA5-S-(CH₂)₂-NH₂ est ensuite purifié par passage sur résine Dowex^{®} 50WX4. L'élution est réalisée avec de l'ammoniaque à 2%. La fraction éluée est concentrée à l'évaporateur rotatif sous pression réduite à sec. Le produit est repris avec 5 mL de méthanol et précipité avec 100 mL de THF. Le précipité est récupéré par filtration. Le produit est re-solubilisé à nouveau dans 5 mL de méthanol et précipité avec 100 mL de THF. On récupère le précipité par filtration, on le reprend dans de l'eau désionisée et le concentre à l'évaporateur rotatif et le lyophilise. On obtient par cette méthode 465 mg de 4GrA5-S-(CH₂)₂-NH₂. La spectrométrie de masse MALDI TOF permet de déterminer une masse attendue de 849 g/mol, c'est-à-dire sous sa forme complexée au sodium 872 g/mol. Le profil 1H RMN était conforme à la structure. La 1H RMN quantitative avec étalonnage interne permet d'estimer la pureté à 100% +/- 3,3%.

### 5) Couplage du 4GrA5-S-(CH₂)₂-NH₂ sur la cellulose activée

On utilise une cellulose micronisée préalablement activée comme décrit à l'exemple 1, caractérisée avec 1 fonction aldéhyde pour 12 glucoses.

Le greffage du 4GrA5 aminé sur cette cellulose activée est réalisé de la manière suivante : 20 g de cellulose activée, 7,530 mL d'une solution de 4GrA5-S-(CH₂)₂-NH₂dans l'eau à 50 g/L, 4,8 mL de tampon phosphate 0,5M pH3, 69,270 mL d'eau et 20 mL d'une solution de NaCNBH₃ à 10g/L sont mélangés et agités environ 72h à température ambiante. La cellulose greffée obtenue est ensuite abondamment rincée à l'eau, puis à l'éthanol pur et enfin séchée sous pression réduite. On obtient 18,5g de cellulose greffée.

Le taux de greffage de 4GrA5 aminé est évalué selon la méthode détaillée à l'exemple 1 et permet de déterminer une quantité greffée de 4GrA5 aminé de 16,4 mg/g de support final, correspondant à une quantité de 4GrA5 (après soustraction de la masse correspondant au bras de liaison) greffée de 10,8 mg/g de support final. Ceci correspond à 314 moles d'unités glucose de la cellulose pour 1 mole d'oligosaccharide greffé. Le taux d'aldéhydes résiduels après greffage est déterminé selon la méthode décrite à l'exemple 1, il est ici de 1 pour 15 unités glucose.

Le ratio nombre de molécules d'oligosaccharide(s) greffées sur la phase solide/nombre de fonctions aldéhyde libres présentes sur la phase solide est égal à 1/26.

### Exemple 4 : Préparation de 4GrB5-S-(CH₂)₂-NH₂ et son greffage, sur de la cellulose micronisée préalablement activée

### 1) Production du 4GrB5-Allyle par fermentation

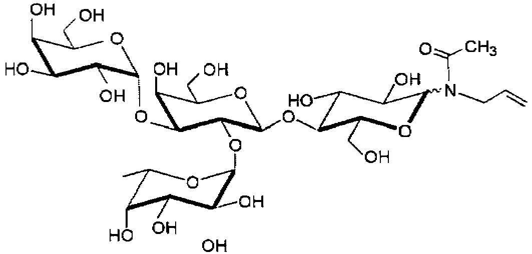

La production du 4GrB5-Allyle est réalisée en fournissant à une souche d'E. Coli modifiée du Lactose Allyle préparé précédemment (procédé décrit dans le brevet WO 2001004341 A1). La souche productrice de 4GrB5 est référencée SD107 à Elicityl (Randriantsoa, m. (2008). Synthèse Microbiologique des antigènes glucidiques des groupes sanguins. Thèse de l'Université Joseph Fourrier Grenoble). On procède comme décrit à l'exemple 3-1).

### 2) Purification du 4GrB5-Allyle produit par fermentation

La purification est réalisée comme décrit à l'exemple 3-2). On obtient par cette méthode de purification 6,8 g de 4GrB5-Allyle. L'identité et la structure de l'oligosaccharide sont validées en spectrométrie de masse MALDI TOF et RMN du proton. La spectrométrie de masse MALDI TOF permet de déterminer une masse attendue de 731 g/mol, c'est-à-dire sous sa forme complexée au sodium 754 g/mol. Le profil 1H RMN était conforme à la structure. La 1H RMN quantitative avec étalonnage interne permet d'estimer la pureté à 100% +/- 3,8%.

### 3) Activation du 4GrB5-Allyle par introduction d'une fonction amine

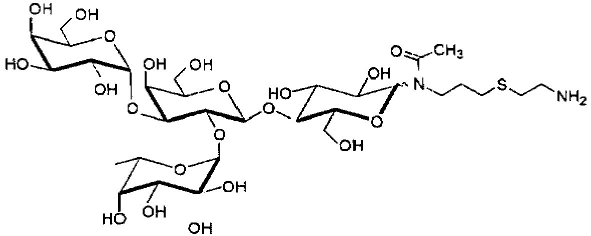

Le 4GrB5-Allyle obtenu précédemment est aminé par réaction de la fonction allyle avec la fonction thiol de la cystéamine. Pour cela, on mélange 500 mg de 4GrB5-Allyl produit précédemment avec 699 mg de cystéamine et 17,2 mg d'acide 4-cyanovalérique dans 5 mL d'eau déionisée. La solution est maintenue trois heures sous agitation à 80°C. Pour le reste, on procède comme décrit à l'exemple 3-3). On obtient par cette méthode 461 mg de 4GrA5-S-(CH₂)₂-NH₂. L'identité et la structure de l'oligosaccharide sont validées en spectrométrie de masse MALDI TOF et RMN du proton. La spectrométrie de masse MALDI TOF laisse apparaître la masse attendue de 808, c'est-à-dire sous sa forme complexée au sodium 831. Le profil RMN était conforme à la structure. La RMN quantitative avec étalonnage interne permet d'estimer la pureté à 97% +/- 4,9%.

### 4) Couplage du 4GrB5-S-(CH₂)₂-NH₂ sur la cellulose activée

On utilise une cellulose micronisée préalablement activée, comme décrit à l'exemple 1, caractérisée avec 1 fonction aldéhyde pour 12 glucoses.

Le greffage du 4GrB5 aminé sur cette cellulose activée est réalisé comme à l'exemple 3-4), en utilisant 7,166 mL d'une solution de 4GrB5-S-(CH₂)₂-NH₂ dans l'eau à 50 g/L. On obtient 18,9 g de cellulose greffée.

Le taux de greffage de 4GrB5 aminé est évalué selon la méthode détaillée à l'exemple 1 et conduit à une quantité greffée de 4GrB5 aminé de 13,5 mg/g de support final, correspondant à une quantité de 4GrB5 (après soustraction de la masse correspondant au bras de liaison) greffé de 9,3 mg/g de support final. Ceci correspond à 364 moles d'unités glucose de la cellulose pour 1 mole d'oligosaccharide greffé. Le taux d'aldéhydes résiduels après greffage est déterminé selon la méthode décrite à l'exemple 1, il est ici de 1 pour 16 unités glucose.

Le ratio nombre de molécules d'oligosaccharide(s) greffées sur la phase solide/nombre de fonctions aldéhyde libres présentes sur la phase solide est égal à 1/27.

### Exemples comparatifs 1 et 2 - Réduction des fonctions aldéhyde résiduelles présentes sur les supports des exemples 1 et 2

On met en suspension 13g du support cellulosique greffé de l'exemple 1 ou 13g du support cellulosique greffé de l'exemple 2 avec 100 mL d'eau désionisée et 300 mg de NaBH₄ conduisant à la réduction des fonctions aldéhyde en fonctions alcool. On agite 2h à température ambiante. On rince ensuite 4 fois avec 100 mL d'eau désionisée, puis 3 fois avec 100 mL d'éthanol. La poudre obtenue est séchée à l'étuve à 50°C pendant 24h. On obtient respectivement 12,4 g de l'exemple 1 réduit et 12,8 g de l'exemple 2 réduit.

Le dosage des fonctions aldéhyde libres est réalisé suivant la méthode du dosage au DNS décrite à l'exemple 1. Le taux de greffage est déterminé suivant la méthode de dosage des monosaccharides en HPAEC avec hydrolyse totale, décrite précédemment.

**Tableau 2**

| Echantillon | Fonction aldéhyde | 6GrA1 Aminé* | ratio oligo de la phase solide/nombre de fonctions aldéhyde libres sur la phase solide |
|---|---|---|---|
| Exemple 1 | 1 aldéhyde pour 18 glucoses | 12,0 mg/g | 1/38 |
| Exemple 1 *(après réduction)* | 1 aldéhyde pour 327 glucoses | 11,5 mg/g | 1/2 |

| Echantillon | Fonctions aldéhyde | 6GrB1 Aminé* | ratio oligo de la phase solide/nombre de fonctions aldéhyde libres sur la phase solide |
|---|---|---|---|
| Exemple 2 | 1 aldéhyde pour 19 glucoses | 12,9 mg/g | 1/33 |
| Exemple 2 *(après réduction)* | 1 aldéhyde pour 391 glucoses | 11,8 mg/g | 1/2 |

| | | | |
|---|---|---|---|
| ^{∗} mg d'oligosaccharide aminé (6GrA1 aminé ou 6GrB1 aminé)/g de support | | | |

### Exemples comparatifs 3 et 4 - Réduction des aldéhydes résiduels sur les supports décrits aux exemples 3 et 4

On met en suspension 1 g du support cellulosique greffé de l'exemple 3 ou du support cellulosique greffé de l'exemple 4 avec 5 mL de tampon phosphate 2M pH 7, 5 mL d'eau désionisée et 156 mg de NaBH₄. On agite 30 mn à température ambiante. On rince ensuite 4 fois avec 100 mL d'eau désionisée, puis 3 fois avec 100 mL d'éthanol. La poudre obtenue est séchée sous pression réduite à température ambiante pendant 72h. On obtient respectivement 870 mg d'exemple comparatif 3 (exemple 3 avec fonction aldéhyde résiduelle sous forme réduite) et 670 mg d'exemple comparatif 4 (exemple 4 avec fonction aldéhyde résiduelle sous forme réduite).

Le dosage des fonctions aldéhyde libres est réalisé suivant la méthode du dosage au DNS décrite à l'exemple 1. Le taux de greffage est déterminé suivant la méthode de dosage des monosaccharides en HPAEC avec hydrolyse totale, décrite précédemment.

**Tableau 3**

| Echantillon | Fonctions aldéhyde | 4GrA5 Aminé^{∗} | ratio oligo de la phase solide/nombre de fonctions aldéhyde libres sur la phase solide |
|---|---|---|---|
| Exemple 3 | 1 aldéhyde pour 15 glucoses | 13,1 mg/g | 1/26 |
| Exemple comparatif 3 *(après réduction)* | 1 aldéhyde pour 322 glucoses | 13,2 mg/g | 1/1 |

| Echantillon | Fonctions aldéhyde | 4GrB5 Aminé^{∗} | ratio oligo de la phase solide/nombre de fonctions aldéhyde libres sur la phase solide |
|---|---|---|---|
| Exemple 4 | 1 aldéhyde pour 16 glucoses | 11,7 mg/g | 1/27 |
| Exemple comparatif 4 *(après réduction)* | 1 aldéhyde pour 284 glucoses | 11,6 mg/g | 1/2 |

| | | | |
|---|---|---|---|
| ^{∗}mg d'oligosaccharide aminé (4GrA5 aminé ou 4GrB5 aminé) /g de support | | | |

### Exemple 5 - Activation de la cellulose microcristalline et greffage de l'oligosaccharide 6GrA1-NAc Propargyl préalablement activé avec une fonction amine.

De la cellulose microcristalline, Vivapur^{®} type 101 produite par Rettenmaier, est activée par la création de fonctions aldéhyde, par traitement au métaperiodate, NaIO₄. Pour cela, on mélange 30 g de cellulose microcristalline avec 6g de NaIO₄ et 300mL d'eau. Le mélange est maintenu pendant 12 heures sous agitation à température ambiante. Le support cellulosique obtenu est rincé à l'eau, puis à l'éthanol et séché à l'étuve à environ 50°C.

Le nombre de fonctions aldéhyde ainsi créées sur le support est mesuré par dosage colorimétrique des fonctions réductrices, conformément à l'exemple 1 et donne 1 fonction aldéhyde pour 17 unités glucose.

L'oligosaccharide 6GrA1-NAc-Propargyl activé par introduction d'une fonction amine (nommé 6GrA1 aminé), tel que décrit à l'exemple 1, est utilisé pour le greffage.

Le greffage du 6GrA1 aminé sur le support cellulosique activé précédemment est réalisé comme à l'exemple 1.

Le taux de greffage de 6GrA1 aminé est évalué selon la méthode détaillée à l'exemple 1 et conduit à une quantité greffée de 6GrA1 aminé de 9,4 mg/g de support final, correspondant à une quantité de 6GrB1 (après soustraction de la masse correspondant au bras de liaison) greffée de 7,3 mg/g de support final. Ceci correspond à 880 moles d'unités glucose de la cellulose pour 1 mole d'oligosaccharide greffé. Le taux d'aldéhydes résiduels après greffage est déterminé selon la méthode décrite à l'exemple 1 et est ici de 1 pour 18 unités glucose.

Le ratio nombre de molécules d'oligosaccharide(s) greffées sur la phase solide/nombre de fonctions aldéhyde libres présentes sur la phase solide est égal à 1/49.

### Exemple 6 - Activation de la cellulose microcristalline et greffage de l'oligosaccharide 6GrB1-NAc Propargyl préalablement activé avec une fonction amine.

On utilise une cellulose microcristalline préalablement activée comme décrit à l'exemple 5, caractérisée avec 1 fonction aldéhyde pour 17 glucoses.

L'oligosaccharide 6GrB1-NAc-Propargyl activé par introduction d'une fonction amine (nommé 6GrB1 aminé), tel que décrit à l'exemple 2, est utilisé pour le greffage.

Le greffage du 6GrB1 aminé sur le support cellulosique activé comme à l'exemple 1.

Le taux de greffage de 6GrB1 aminé est évalué selon la méthode détaillée à l'exemple 1 et conduit à une quantité greffée de 6GrB1 aminé de 10,3 mg/g de support final, correspondant à une quantité de 6GrB1 (après soustraction de la masse correspondant au bras de liaison) greffé A de 8,0 mg/g de support final. Ceci correspond à 779 moles d'unités glucose de la cellulose pour 1 mole d'oligosaccharide greffé. Le taux d'aldéhydes résiduels après greffage est déterminé selon la méthode décrite à l'exemple 1, il est ici de 1 pour 18 unités glucose.

Le ratio nombre de molécules d'oligosaccharide(s) greffées sur la phase solide/nombre de fonctions aldéhyde libres présentes sur la phase solide est égal à 1/43.

### Exemples 7 et 8 - Production de supports greffés à différentes concentration de 6 GRA1 et GRB1 :

On utilise la cellulose micronisée préalablement activée, produite à l'exemple 1, comprenant 1 fonction aldéhyde pour 14 unités glucose.

On utilise également les oligosaccharides préalablement activés, produits respectivement à l'exemple 1 pour le 6GrA1 aminé et à l'exemple 2 pour le 6GrB1 aminé.

Le greffage du 6GrA1 aminé et du 6GrB1 aminé sur la cellulose micronisée activée est réalisé respectivement comme aux exemples 1 et 2, à l'exception de la quantité d'oligosaccharide amine appliquée qui correspond à celle présentée dans le **Tableau 4** ci-après :

**Tableau 4**

| | Cellulose 6GrA1 | | | Cellulose 6GrB1 | | |
|---|---|---|---|---|---|---|
| | Ex. 7A | Ex. 7B | Ex. 7C | Ex. 8A | Ex. 8B | Ex. 8C |
| | | | | | | |
| Cellulose micronisée (g) | 1,25 | 1,25 | 1,25 | 1,25 | 1,25 | 1,25 |
| 6GrA1 Aminé à 50mg/ml (µL) | 500 | 250 | 125 | - | - | - |
| 6GrB1 Aminé à 50mg/ml (µL) | - | - | - | 500 | 250 | 125 |
| Tampon Phosphate 0,5M pH3 (µL) | 200 | 200 | 200 | 200 | 200 | 200 |
| eau désionisée (µL) | 3050 | 3300 | 3425 | 3050 | 3300 | 3425 |
| | | | | | | |
| Agitation environ 16 h à température ambiante | | | | | | |
| | | | | | | |
| NaCNBH₃ à 100g/L (µL) | 1250 | 1250 | 1250 | 1250 | 1250 | 1250 |
| | | | | | | |
| Agitation environ 8 h à température ambiante | | | | | | |
| | | | | | | |
| Le support greffé est ensuite abondamment rincé à l'eau, puis à l'éthanol pur et est ensuite séché à une température de l'ordre de 50°C. | | | | | | |

Le taux de greffage de 6GrA1 aminé et 6GrB1 aminé est évalué selon la méthode détaillée à l'exemple 1 et conduit aux quantités greffées de 6GrA1 aminé et 6GrB1 aminé suivantes :

**Tableau 5 -**

| Supports greffés | Exemple | mg oligo aminé/g de support ^{∗ ∗} | mg d'oligo/g de support^{∗∗} | ratio oligo de la phase solide/nombre de fonctions aldéhyde libres sur la phase solide |
|---|---|---|---|---|
| | | | | |
| Cellulose micronisée 6GrA1 | Ex.7A | 9,1 | 7,1 | 1/57 |
| | Ex.7B | 5,7 | 4,5 | 1/91 |
| | Ex.7C | 3,3 | 2,6 | 1/158 |
| Cellulose micronisée 6GrB1 | Ex.8A | 8,6 | 6,7 | 1/58 |
| | Ex.8B | 5,6 | 4,3 | 1/90 |
| | Ex.8C | 3,7 | 2,9 | 1/136 |

| | | | | |
|---|---|---|---|---|
| ^{∗} mg d'oligosaccharide aminé (6GrA1 aminé ou 6GrB1 aminé) /g de support final ^{∗∗} mg d'oligosaccharide (6GrA1 ou 6GrB1 après soustraction de la masse correspondant au bras de liaison) /g de support final | | | | |

**Exemples 9 et 10** - Production de supports greffés avec des oligosaccharides des groupes sanguins A ou B avec variation 1) du taux de fonctions aldéhyde présentes avant greffage et 2) du bras de liaison utilisé pour le greffage de l'oligosaccharide et 3) de la taille de l'oligosaccharide (hexasaccharide de type 1, tetrasaccharide de type 5 ou trisaccharide).

Les celluloses micronisées activées utilisées sont soit activées comme à l'exemple 1 (nommée cellulose haute activation), soit activées comme à l'exemple 1 à l'exception du fait que le métaperiodate est introduit avec une proportion dix fois moins importante (10 g de métaperiodate pour 500g de cellulose). Dans ce cas où la cellulose est activée avec moins de métaperiodate, une cellulose comportant 1 fonction aldéhyde pour 105 glucoses (nommée cellulose basse activation) est obtenue.

Différents oligosaccharides des groupes sanguins greffés commercialisés par Elicityl sont utilisés : 6GrA1-NAc-Propargyl (OligoTech ref GLY037-1-NPR), 6GrB1-NAc-Propargyl (OligoTech ref GLY040-1-NPR), 4GrA5-NAc-Propargyl (OligoTech ref GLY035-3-NPR), 4GrB5-NAc-Propargyl (OligoTech ref GLY038-3-NPR) et 3GrA5-NAc-Propargyl (OligoTech ref GLY031-3-NPR). Chaque oligosaccharide est activé sous forme aminée en respectant les équivalences et le mode opératoire décrit dans l'exemple 1. Seul l'espaceur amine/azide utilisé présente deux variantes : soit on utilise l'espaceur utilisé dans l'exemple 1 (11-azido-3,6,9-trioxaundécan-1-amine - TCI Chemical référence A2363), soit le O-(2-aminoéthyl)-O'-(2-azidoéthyl)heptaéthylène glycol (Aldrich ref 76318).

On obtient alors, dans le cas où l'espaceur utilisé est le 1 (11-azido-3,6,9-trioxaundécan-1-amine:
- Pour 6GrA1, l'oligosaccharide aminé suivant : 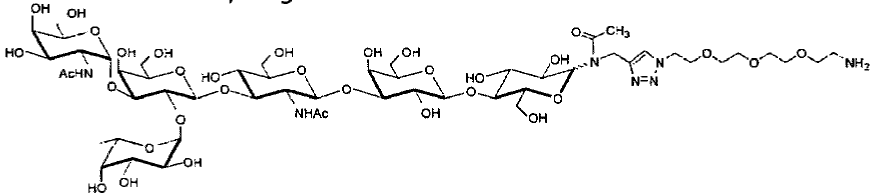
- Pour 6GrB1 l'oligsaccharide aminé suivant : 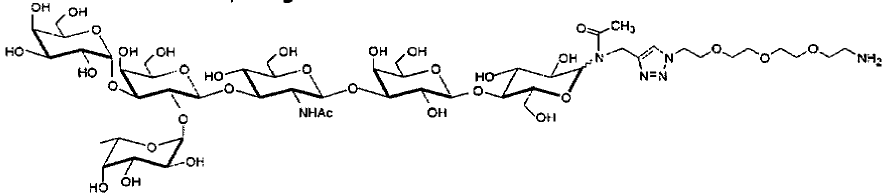
- Pour 4GrA5 l'oliaosaccharide aminé suivant : 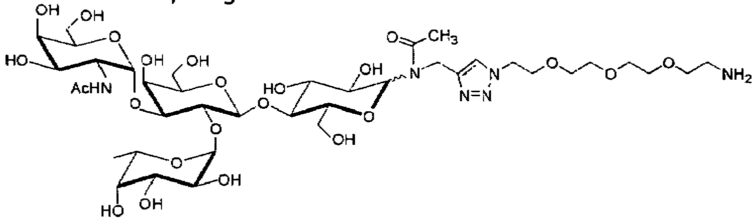
- Pour 4GrB5, l'oligosaccharide aminé suivant : 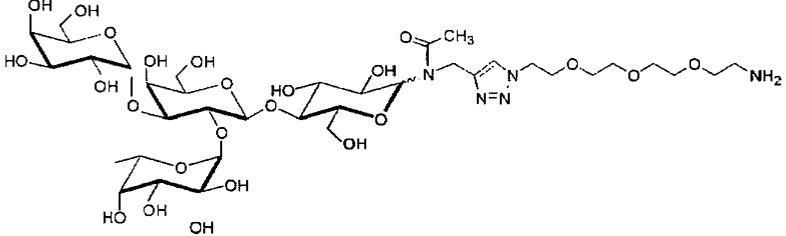
- Pour 3GrA, l'oligosaccharide aminé suivant :

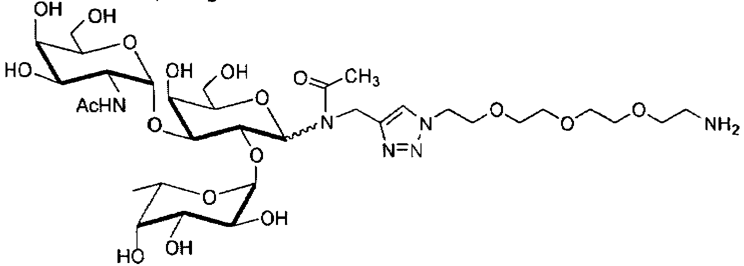

On obtient alors, dans le cas où l'espaceur utilisé est le O-(2-aminoéthyl)-O'-(2-azidoéthyl)heptaéthylène glycol :
- Pour 6GrA1 l'oligosaccharide aminé suivant : 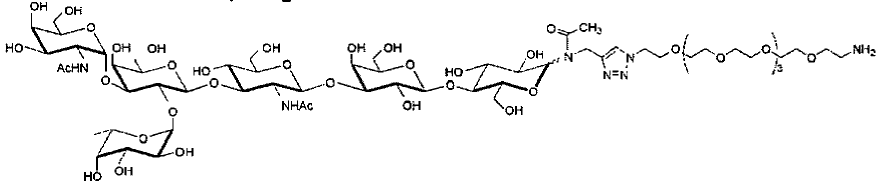
- Pour 6GrB1, l'oligosaccharide aminé suivant : 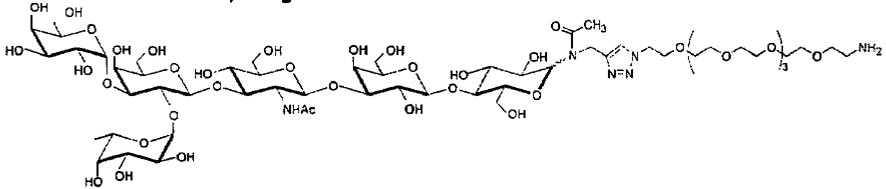
- Pour 4GrA5 l'oligosaccharide aminé suivant : 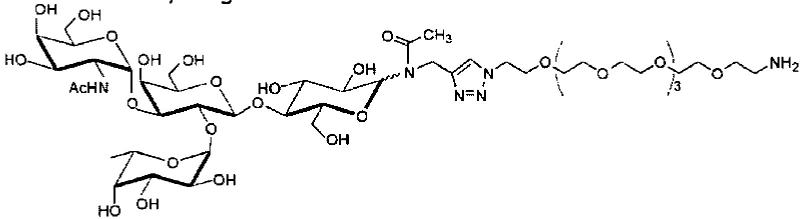
- Pour 4GrB5 l'oligsaccharide aminé suivant : 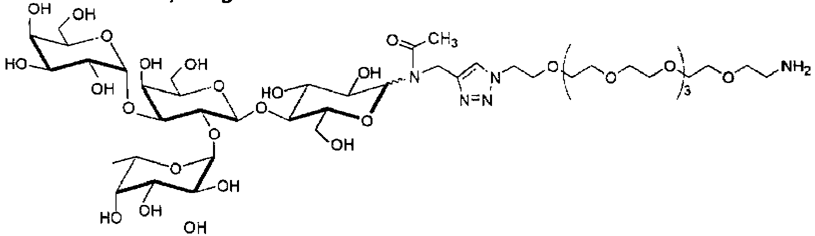
- Pour 3GrA, l'oligosaccharide aminé suivant :

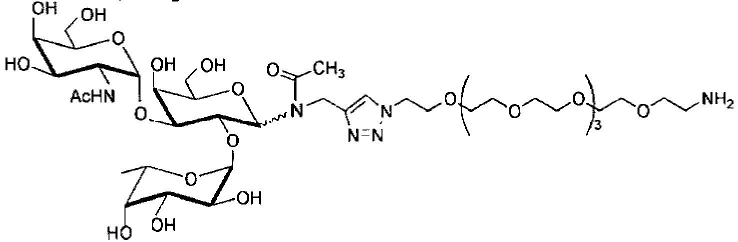

Les différents essais réalisés sont résumés dans le **Tableau 6** ci-après :

**Tableau 6**

| **Oligosaccharide aminé préparé** | **Réactifs utilisés pour la click chemistry** | |
|---|---|---|
| | **Ref de l'Oligo** | **Espaceur** |
| | | |
| 6GrA1-Sp1-NH₂ | GLY037-1-NPR | 11-azido-3,6,9-trioxaundécan-1-amine |
| 6GrB1-Sp1-NH₂ | GLY040-1-NPR | 11-azido-3,6,9-trioxaundécan-1-amine |
| 6GrA1-Sp2-NH₂ | GLY037-1-NPR | O-(2-aminoéthyl)-O'-(2- |
| | | azidoéthyl)heptaéthylène glycol |
| 6GrB1-Sp2-NH₂ | GLY040-1-NPR | O-(2-aminoéthyl)-O'-(2- |
| | | azidoéthyl)heptaéthylène glycol |
| 4GrA5-Sp1-NH₂ | GLY035-3-NPR | 11-azido-3,6,9-trioxaundécan-1-amine |
| 4GrB5-Sp1-NH₂ | GLY038-3-NPR | 11-azido-3,6,9-trioxaundécan-1-amine |
| 4GrA5-Sp2-NH₂ | GLY035-3-NPR | O-(2-aminoéthyl)-O'-(2- |
| | | azidoéthyl)heptaéthylène glycol |
| 4GrB5-Sp2 -NH₂ | G LY038- 3-N PR | O-(2-Aminoéthyl)-O'-(2- |
| | | azidoéthyl)heptaéthylène glycol |
| 3GrA-Sp1-NH₂ | GLY031-3-NPR | 11-azido-3,6,9-trioxaundécan-1-amine |
| 3GrA-Sp2-NH₂ | GLY031-3-NPR | O-(2-aminoéthyl)-O'-(2- |
| | | azidoéthyl)heptaéthylène glycol |

Chaque oligosaccharide aminé est greffé sur l'une des deux celluloses activées décrites précédemment suivant le protocole décrit à l'exemple 1 et suivant les **Tableaux 7 et 8** ci-après :

**Tableau 7 - Celluloses greffées avec un oligosaccharide du groupe A**

| | Ex. 9A | Ex. 9B | Ex. 9C | Ex. 9D | Ex. 9E | Ex. 9F | Ex. 9G |
|---|---|---|---|---|---|---|---|
| Cellulose micro. | 1,25 | - | 0,8 | 0,8 | 0,8 | 0,8 | 0,8 |
| haute activation (g) | | | | | | | |
| Cellulose micro. | | 0,8 | | | | | |
| basse activation (g) | | | | | | | |
| 6GrA1-Sp1-NH2 | 500 | 320 | | | | | |
| à 50mg/ml (µl) | | | | | | | |
| 6GrA1-Sp2-NH2 | | | | | 372 | | |
| à 50mg/ml (µl) | | | | | | | |
| 4GrA5-Sp1-NH2 | | | 234 | | | | |
| à 50mg/ml (µl) | | | | | | | |
| 4GrA5-Sp2-NH2 | | | | | | 263 | |
| à 50mg/ml (µl) | | | | | | | |
| 3GrA5-Sp1-NH2 | | | | 195 | | | |
| à 50mg/ml (µl) | | | | | | | |
| 3GrA5-Sp2-NH2 | | | | | | | 225 |
| à 50mg/ml (µl) | | | | | | | |
| Tampon Phosphate | 200 | 128 | 128 | 128 | 128 | 128 | 128 |
| 0,5M pH3 (µl) | | | | | | | |
| eau désionisée (µl) | 3050 | 1952 | 2038 | 2077 | 1900 | 2009 | 2047 |
| | | | | | | | |
| Agitation environ 16 h à température ambiante | | | | | | | |
| | | | | | | | |
| NaCNBH₃ à 100g/L (µl) | 1250 | 800 | 800 | 800 | 800 | 800 | 800 |
| | | | | | | | |
| Agitation environ 8 h à température ambiante | | | | | | | |
| | | | | | | | |
| Le support greffé est ensuite abondamment rincé à l'eau, puis à l'éthanol pur et est ensuite séché à une température de l'ordre de 50°C. | | | | | | | |

**Tableau 8 - Celluloses greffées avec un oligosaccharide du groupe B**

| | Ex. 10A | Ex. 10B | Ex. 10C | Ex. 10D |
|---|---|---|---|---|
| Cellulose micro. haute activation (g) | 1,25 | - | 0,8 | 0,8 |
| Cellulose micro. basse activation (g) | | 0,8 | | |
| 6GrB1-Sp1-NH2 à 50mg/ml (µl) | 500 | 320 | | |
| 6GrB1-Sp2-NH2 à 50mg/ml (µl) | | | | 374 |
| 4GrB5-Sp1-NH2 à 50mg/ml (µl) | | | 231 | |
| Tampon Phosphate 0,5M pH3 (µl) | 200 | 128 | 128 | 128 |
| eau désionisée (µl) | 3050 | 1952 | 2041 | 1898 |
| | | | | |
| Agitation environ 16 h à température ambiante | | | | |
| | | | | |
| NaCNBH₃ à 100g/L (µl) | 1250 | 800 | 800 | 800 |
| | | | | |
| Agitation environ 8 h à température ambiante | | | | |
| | | | | |
| Le support greffé est ensuite abondamment rincé à l'eau, puis à l'éthanol pur et est ensuite séché à une température de l'ordre de 50°C. | | | | |

Le taux de greffage d'oligosaccharides est évalué selon la méthode détaillée à l'exemple 1 et conduit aux quantités greffées d'oligosaccharides suivantes :

| **Supports greffés** | **Exemple** | **mg oligo aminé /g de support ^{∗}** | mg oligo/g de support | ratio oligo de la phase solide/nombre de fonctions aldéhyde libres sur la phase solide |
|---|---|---|---|---|
| | | | | |
| | Ex. 9A | 9,1 | 7,1 | 1/53 |
| | Ex. 9B | 3,4 | 2,7 | 1/22 |
| Cellulose greffée | Ex. 9C | 9,2 | 6,4 | 1/39 |
| avec oligo du | Ex. 9D | 11,5 | 7,4 | 1/26 |
| Groupe A | Ex. 9E | 6,7 | 4,5 | 1/85 |
| | Ex. 9F | 9,0 | 5,6 | 1/44 |
| | Ex. 9G | 8,1 | 4,5 | 1/42 |
| | Ex. 10A | 8,6 | 6,7 | 1/55 |
| Cellulose greffée | Ex. 10B | 4,4 | 3,4 | 1/16 |
| avec oligo du | Ex. 10C | 8,9 | 6,5 | 1/38 |
| Groupe B | Ex. 10D | 6,2 | 4,1 | 1/89 |

| | | | | |
|---|---|---|---|---|
| ^{∗} mg d'oligosaccharide concerné /g de support final ^{∗∗} mg d'oligosaccharide concerné (après soustraction de la masse correspondant au bras de liaison) /g de support final | | | | |

### Exemple 11 et exemple comparatif 11 - Billes Tosoh réduites et non réduites après couplage d'oligosaccharides 4GRA5

Le greffage de l'oligosaccharide 4GrA5 aminé sur le support activé chromatographique macroporeux Tosoh Biosciences (650 AF Formyl - polymère méthacrylique) présentant des fonctions aldéhyde de surface est réalisé de la manière suivante :
3 ml de support Tosoh Biosciences (650 AF Formyl), 18,9 mg de 4GrA5 aminé précédemment préparé à l'exemple 3 et repris dans 378 µL d'eau désionisée, 1,5 mL de tampon phosphate 0,5M pH3 et 10 M de NaCNBH₃ sont mélangés 66 h environ à 25°C. Le support greffé est ensuite abondamment rincé à l'eau. 1,5 mL du gel sont conservés pour constituer la fraction non réduite (exemple 11). Par ailleurs, 1,5 ml du même gel greffé est réduit par NaBH₄ à la concentration de 3mg/ mL pendant 1h. L'opération est renouvelée une fois avec 3mg/mL de NaBH₄ une 1 h. Le gel est ensuite abondement rincé à l'eau et 1,5 mL du gel sont conservés pour constituer la fraction réduite (exemple comparatif 11).

Le taux de fonction aldéhyde est mesuré suivant la méthode de dosage au DNS décrite dans l'exemple 1. Le dosage est réalisé suivant une calibration au glucose exprimée en µmol d'aldéhyde/ml, sachant que chaque glucose comporte une fonction aldéhydique. Les taux mesurés sont les suivants :

| | | | | |
|---|---|---|---|---|
| Gel non réduit (Ex. 11) | | 13,5 µmol/ml | | |
| Gel réduit (Ex. comparatif 11) | | 1,4 µmol/ml | | |

| Echantillon | Fonctions aldéhyde | | 4GrA5 Aminé^{∗} | ratio oligo de la phase solide/nombre de fonctions aldéhyde libres sur la phase solide |
|---|---|---|---|---|
| Exemple 11 | 13,5 µmol/ml | | 3,2 µmol/ml | 1/4 |
| Exemple 11 *(après réduction)* | 1,4 µmol/ml | | 3,1 µmol/ml | 1/1 |

| | | | | |
|---|---|---|---|---|
| ^{∗} en mg d'oligosaccharide concerné/g de support final | | | | |

### Exemple 12 : greffage sur cellulose Ultrafine

### Activation de cellulose Ultrafine

De la cellulose Ultrafine, Vitacel UFC 100, produite par Rettenmaier et ayant des taille de particules ellipsoïdales d'approximativement 2,2 µm X 8 µm, est activée par la création de fonctions aldéhyde, obtenues par un traitement au métaperiodate NaIO₄. Pour cela, on mélange 150 g de cellulose micronisée avec 30 g de NaIO₄ et 1,5 L d'eau. Le mélange est maintenu à température ambiante pendant 12 h, sous agitation, puis le support cellulosique obtenu est rincé 4 fois par 3 L d'eau, puis 3 fois par 1,5 L d'éthanol et séché à l'étuve à une température d'environ 50°C.

### Couplage de l'oligosaccharide 4GrA5-S-(CH₂)₂-NH₂ sur la cellulose activée

On utilise la cellulose Ultrafine activée comme décrit précédemment. Le greffage de l'oligosaccharide 4GrA5 aminé sur cette cellulose activée est réalisé de la manière suivante : 15 g de cellulose activée, 5,648 mL d'une solution de 4GrA5-S-(CH₂)₂-NH₂ dans l'eau à 50 mg/mL, 3,6 mL de tampon phosphate 0,5 M pH 3, 51,953 mL d'eau désionisée et 15 mL d'une solution de NaCNBH₃ à 10 mg/mL sont mélangés et agités environ 72 h à température ambiante. La cellulose greffée obtenue est ensuite abondamment rincée par 2 fois 200 mL d'eau, puis par 2 fois 200 mL d'éthanol pur et enfin séchée sous pression réduite. On obtient 12,48 g de cellulose greffée **(support A).**

Le taux de greffage de 4GrA5 aminé est évalué selon la méthode détaillée à l'exemple 1 et permet de déterminer une quantité greffée de 4GrA5 aminé de 15,08 mg/g de support final, correspondant à une quantité de 4GrA5 (après soustraction de la masse correspondant au bras de liaison) greffée de 9,93 mg/g de support final. Ceci correspond à 341 moles d'unités glucose de la cellulose pour 1 mole d'oligosaccharide greffé. Le taux de fonctions aldéhyde libres résiduelles après greffage est déterminé selon la méthode décrite à l'exemple 1, il est ici de 1 pour 18 unités glucose.

Le ratio nombre de molécules d'oligosaccharide(s) greffées sur la phase solide/nombre de fonctions aldéhyde libres présentes sur la phase solide est égal à 1/19.

### Couplage de l'oligosaccharide 4GrB5-S-(CH₂)₂-NH₂ sur la cellulose activée

On utilise la cellulose Ultrafine activée comme décrit précédemment. Le greffage de l'oligosaccharide 4GrB5 aminé sur cette cellulose activée est réalisé de la manière suivante : 15 g de cellulose activée, 5,375 mL d'une solution de 4GrB5-S-(CH₂)₂-NH₂ dans l'eau à 50 mg/mL, 3,6 mL de tampon phosphate 0,5 M pH 3, 51,953 mL d'eau désionisée et 15 mL d'une solution de NaCNBH₃ à 10 mg/mL sont mélangés et agités environ 72 h à température ambiante. La cellulose greffée obtenue est ensuite abondamment rincée par 2 fois 200 mL d'eau, puis par 2 fois 200 mL d'éthanol pur et enfin séchée sous pression réduite. On obtient 14,18 g de cellulose greffée **(Support B).**

Le taux de greffage de 4GrB5 aminé est évalué selon la méthode détaillée à l'exemple 1 et permet de déterminer une quantité greffée de 4GrB5 aminé de 16,55 mg/g de support final, correspondant à une quantité de 4GrB5 (après soustraction de la masse correspondant au bras de liaison) greffée de 11,40 mg/g de support final. Ceci correspond à 296 moles d'unités glucose de la cellulose pour 1 mole d'oligosaccharide greffé. Le taux fonctions aldéhyde libres résiduelles après greffage est déterminé selon la méthode décrite à l'exemple 1, il est ici de 1 pour 18 unités glucose.

Le ratio nombre de molécules d'oligosaccharide(s) greffées sur la phase solide/nombre de fonctions aldéhyde libres présentes sur la phase solide est égal à 1/16.

### Exemple comparatif 12: Réduction des fonctions aldéhyde résiduelles sur le support greffé avec l'oligosaccharide 4GrA5-S-(CH₂)2-NH₂ décrit à l'exemple 12

On met en suspension 200 mg du support cellulosique greffé de l'exemple 12 (support A ou support B) avec 250 µL de tampon phosphate 2 M pH 8,2 et 750 µL d'eau désionisée contenant 8,26 mg de NaBH₄. On agite 30 min à température ambiante. On rince ensuite 3 fois avec 80 mL d'eau désionisée, puis 2 fois avec 25 mL d'éthanol. La poudre obtenue est séchée sous pression réduite à 50°C pendant 4 h puis 1 h sous pression réduite à 22°C. On obtient 189,8 mg de support greffé et réduit. Le dosage des fonctions aldéhyde libres est réalisé suivant la méthode du dosage au DNS décrite à l'exemple 1. Le résultat est de 1 aldéhyde pour 262 glucoses, soit statistiquement un nombre voisin du nombre d'unités glucose par oligosaccharide greffé. Le ratio du nombre d'oligosaccharides fixés sur la phase solide par rapport au nombre de fonctions aldéhyde libres sur cette phase solide est (1/341)/ (1/262) soit 0,77.

### II. EVALUATION DES SUPPORTS

Les plasmas avant et après épuration sont testés selon deux méthodes :
1-La méthode d'hémagglutination, méthode de Simonin, référence règlementaire, qui teste le pouvoir agglutinant des anticorps sur des hématies humaines du groupe à tester (A ou B). Cette méthode est surtout sensible aux IgM pentamériques (10 sites d'affinité) par rapport aux IgG monomériques (deux fonctions réactives).
2-La méthode du gel test (Lapierre Y., Rigal D., Adam J., Josef D., Meyer F., Greber S., Drot C. Transfusion. 1990 Feb ;30(2) :109-13) qui utilise une colonne de gel miniaturisée contenant des anticorps anti IgG, après mise en contact des hématies avec le plasma. Cette méthode permet une quantification du titre en anticorps de type IgG et IgM. Elle est particulièrement intéressante pour le titrage des IgG.

Les échantillons de plasma, avant ou après épuration sont dilués en tampon PBS, par dilution successive d'un facteur 2 (1/1, 1/2.....1/ 4096) et mis en contact avec des hématies du groupe sanguin considérées diluées 1/10 dans le même tampon.

Le titre rendu correspond à la dernière dilution positive.

**A.** Les supports greffés avec les oligosaccharides 6GRA1 et 6GrB1 sont comparés, quant à leur efficacité pour l'épuration plasmatique :
3mL d'un plasma positif au 1/32 en anticorps anti A et au 1/32 en anticorps anti B sont mis en contact avec 100 mg soit des supports des exemples 1 et 2 (cellulose micronisée), 5 et 6 (cellulose microcristalline), pendant 30 minutes sous agitation. Le test étant réalisé avec un mélange de 50 mg de support A et 50 mg de support B soit respectivement : support 1 et 2 mélangés pour la cellulose micronisée et 5 et 6 pour la cellulose microcristalline. L'épuration est totale pour l'ensemble des supports lorsque l'on pratique le test d'hémagglutination.

Les résultats montrent que les oligosaccharides sélectionnés sont efficaces sur les différents supports cellulosiques sélectionnés pour l'épuration plasmatique.

Le même essai est reproduit avec 4 mL du même plasma et 100 mg des supports des exemples 1, 2, 5 et 6. Dans ce cas, seuls les supports des exemples 1 et 2 montrent une absorption totale du plasma testé.

**B.** Les supports cellulosiques greffés de l'exemple 1 (fonctions aldéhyde résiduelles sous forme non réduite) et de l'exemple comparatif 1 (fonctions aldéhyde résiduelles sous leur forme réduite), d'une part, et de l'exemple 2 (fonctions aldéhyde résiduelles sous forme non réduite) et de l'exemple comparatif 2 (fonctions aldéhyde résiduelles sous leur forme réduite), d'autre part, sont comparés.

Les tests sont réalisés avec un plasma titrant 1/128 en anticorps anti A et 1/128 en anticorps anti B, avec 50 mg de support greffé (25 mg de gel pour la capture des anticorps anti-A et 25 mg de gel pour la capture des anticorps anti-B) pour 1 mL de plasma sous agitation pendant 30 minutes.

Les supports des exemples 1 et 2 (fonctions aldéhyde résiduelles sous forme non réduite) permettent une absorption totale du plasma (en anticorps anti A et en anticorps anti B) avec un titre résiduel non détectable par le gel test et par le test d'hémagglutination. Il y a donc une bonne élimination des IgM sur les deux supports pour ce plasma.

Les supports des exemples comparatifs 1 et 2 (fonctions aldéhyde résiduelles sous leur forme réduite), testés dans les mêmes conditions, donnent chacun une épuration partielle pour les IgG, avec un titre résiduel de 1/32 en gel test. Il apparaît donc que les supports selon l'invention portant des aldéhydes libres sont plus efficaces pour l'épuration des anticorps, en particulier pour les IgG.

**C.** Les supports cellulosiques greffés de l'exemple 3 (fonctions aldéhyde résiduelles sous forme non réduite) et de l'exemple comparatif 3 (fonctions aldéhyde résiduelles sous leur forme réduite), d'une part, et de l'exemple 4 (fonctions aldéhyde résiduelles sous forme non réduite) et de l'exemple comparatif 4 (fonctions aldéhyde résiduelles sous leur forme réduite), d'autre part, sont comparés.

Les tests sont réalisés avec un plasma de titre 1/1024 en anticorps anti A et 1/512 en anticorps anti B comme suit :
10 mg de support cellulosique greffé de l'exemple 3 et 10 mg de support de l'exemple 4, d'une part, et 10 mg de support cellulosique greffé de l'exemple comparatif 3 et 10 mg de support de l'exemple comparatif 4, d'autre part, sont incubés pendant 30 minutes à 22°C sous agitation permanente en tube d'hémolyse.

Les résultats sont les suivants en gel test (anti IgG et anti IgM détectés) :

| Titre résiduel | anticorps anti A | anticorps anti B |
|---|---|---|
| Support des exemples 3 et 4 | 1/ 16 | 1/ 16 |
| Support réduit des exemples comparatifs 3 et 4 | 1/ 64 | 1/64 |

Il apparaît que là encore, la réduction du support s'accompagne d'une moindre efficacité pour l'élimination des anticorps, surtout pour les anticorps de type IgG.

### D. Résultats d'hémostase :

Trois plasmas de groupes respectifs O, A et B fraîchement épurés par absorption sur des supports A et B correspondant aux exemples 1 et 2 sont contrôlés pour leurs qualités hémostatiques Les résultats suivants sont obtenus entre les plasmas contrôle (ctl) et les mêmes plasmas épurés avec les supports en mélange selon l'invention (test) pour leurs anticorps anti groupe A et B :
Les paramètres contrôlés sont les suivants :
FVIII c%: activité coagulante du facteur VIII, mesurée par un essai de coagulation en une étape utilisant le kit bioMérieux-deficient FVIII (bioMérieux, Marcy l'Etoile, France) sur l'instrument MDAII de bioMérieux.
FIXc%: activité coagulante du facteur IX, mesurée par un essai en une étape avec le kit Biophen FactorIX (Hyphen-BioMed, Neuville-sur-Oise, France).
vWF :AG% : antigène du Facteur von Willebrand, test effectué avec les réactifs vWF AG sur un appareil BCS coagulation Analyser (Dade Behring, Marburg, Allemagne).
vWF:Rco% : activité du Facteur von Willebrand, test effectué avec les réactifs BC vWF sur un appareil Dade Behring.
FII% : taux du Facteur II plasmatique ou prothrombine mesuré par un essai en une étape avec un kit utilisant un plasma déficient (STA deficientII, Diagnostica Stago, Asnières-sur-Seine, France).
FVII+X% : taux du facteur VII+X, mesuré par un test en une étape avec un kit utilisant un plasma déficient (Hemoclot VII+X, Hyphen BioMed).
FV% : taux du Facteur V, mesuré par un test en une étape avec un kit utilisant un plasma déficient (Hemoclot Factor V reageant, Hyphen BioMed).

Fibrinogène plasmatique : Fibrinogène, dont la concentration est mesurée par le Fibriquick kit (bioMérieux) basé sur la méthode de Clauss.

| Plasma | FVIIIc% | FIXc% | VWF: AG% | VWF:CO% | FII% | FVII+X% | FV% | Fibrinogène (g/l) |
|---|---|---|---|---|---|---|---|---|
| O test | 80,1 | 99,4 | 114,4 | 95,4 | 73 | 95 | 90 | 1,7 |
| O ctl | 90,1 | 125,4 | 109,2 | 98,4 | 70 | 106 | 107 | 2,5 |
| A test | 46,6 | 100,9 | 62,8 | 63,5 | 92 | 89 | 55 | 2,1 |
| A ctl | 50,5 | 104,1 | 61,4 | 60,7 | 103 | 88 | 56 | 2,5 |
| B test | 36,8 | 78,1 | 46,7 | 43,9 | 97 | 93 | 96 | 1,8 |
| B ctl | 37,6 | 82,9 | 44,5 | 43 | 104 | 93 | 98 | 2,0 |

Ces résultats montrent une très bonne conservation des facteurs de coagulation après l'épuration.

### E. Influence du taux de greffage

Des essais ont été réalisés avec les différents supports greffés de l'exemple 7 à différentes concentration de 6 GRA1.
Les essais ont été réalisés sur deux plasmas O de titres élevés (1/1024 en anticorps anti A et 1/512 en anticorps anti A).

Les incubations sont réalisées avec 50mg de support 7A, 7B ou 7C pour 1ml de plasma, 30mn à 22°C sur agitateur circulaire.
Les plasmas épurés sont ensuite testés en gel test (avec anti IgG) pour déterminer le titre résiduel en IgG et IgM après absorption :

| | | | |
|---|---|---|---|
| Conditions de greffage | Ex. 7C | Ex.7B | Ex.7A |
| Concentrations mg/g de 6GrA1 | 3,3 | 5,7 | 9,1 |

Titres obtenus après épuration :

| | | | |
|---|---|---|---|
| Plasma O de titre initial en anticorps anti A 1/1024 | 1/16 | 1/8 | 0 |
| Plasma O de titre initial en anticorps anti A 1/512 | 1/2 | 0 | 0 |

La concentration d'oligosaccharides greffés pour une masse donnée de support est un facteur déterminant de la qualité de l'épuration.

### F. Essais comparatifs d'épuration en colonne et en batch.

Le protocole d'épuration réalisé en batch peut être réalisé sous un format de colonne chromatographique.

Les supports des exemples 1, 2, 5 et 6 (cellulose micronisée, cellulose microcristalline) testés en batch ont été également mis en œuvre dans un format de colonne chromatographique.

Les supports cellulosiques (cellulose micronisée et cellulose microcristalline) ont montré des comportements chromatographiques très proches.

Les essais ont étés réalisés avec des colonnes de 500 mg de gel pour épurer 5ml de plasma.

Le temps d'écoulement était de 14 minutes +/- 2minutes pour l'ensemble des supports, avec une pression de 0,1 bar.

Les résultats d'épuration sont identiques à ceux obtenus pour le test en batch présentés au paragraphe A.

**G.** Les supports des exemples 3 et 4, porteurs respectivement des oligosaccharides 4GrA5 et 4GrB5 ont été testés dans différentes conditions d'incubation pour des temps et des concentrations différentes.

Le protocole était le suivant :

Essais en poche de plasmaphérèse avec 10ml de plasma (titre en anticorps anti A 1/1024 et titre en anticorps anti B 1/512) avec un mélange contenant la même quantité de supports A et B greffés (50 mg, 100 mg ou 200 mg). Les titres résiduels obtenus étaient les suivants :

| | | | | | | |
|---|---|---|---|---|---|---|
| Quantité de chaque support utilisé | 50 mg | | 100 mg | | 200 mg | |

| | Titre en anticorps anti A | Titre en anticorps anti B | Titre en anticorps anti A | Titre en anticorps anti B | Titre en anticorps anti A | Titre en anticorps anti B |
|---|---|---|---|---|---|---|
| Temps d'incubation 30mn | 1/8 | 1/8 | ¼ | 1/4 | 1/2 | 1/4 |
| Temps d'incubation 60mn | 1/4 | 1/4 | ½ | 1/2 | 1/2 | 0 |
| Temps d'incubation 90mn | 1/4 | 1/2 | ½ | 0 | 0 | 0 |

Ces résultats montrent qu'une incubation de 1h à une concentration de 10 mg/ ml pour chaque support est suffisante pour une épuration des anticorps anti A et anti B très en dessous du seuil à risque, les anticorps résiduels étant des IgG.

### H. Essais d'absorption en phase liquide

Afin d'évaluer la performance des supports préparés, l'effet coopératif des oligosaccharides fixés sur le support et la stabilisation secondaire, nous avons incubé un plasma O de titre élevé (1/1024 en anticorps anti A et 1/512 en anticorps anti B) avec les mêmes oligosaccharides que ceux utilisés fixés sur les supports, mais directement en solution.

Après une heure d'incubation d'un ml de plasma avec des concentrations croissantes d'oligosaccharides tetraoses (4GrA5 et 4GrB5) et hexaoses (6GrA1 et 6GrB1) préalablement dilués en PBS à la concentration de 100 mg/ml (soit 0,1mg/µl), les quantités d'oligosaccharide nécessaires pour la neutralisation complète des anticorps IgG et IgM anti A et B du plasma sont les suivantes :

| Oligosaccharides | 4GrA5 | 4GrB5 | 6GrAl | 6GrB1 |
|---|---|---|---|---|
| Volume de solution PBS utilisée | 60µl | 30µl | 120µl | 60µl |
| Quantité d'oligosaccharide utilisée | 6mg | 3mg | 12mg | 6mg |

Pour des doses plus faibles, une incubation prolongée de 24h ne permet pas une meilleure absorption des anticorps.

A titre comparatif,
- 50mg du support cellulosique de l'exemple 7A portant 0,455 mg d'oligosaccharides 6GrA1 neutralise (c'est-à-dire permet l'obtention d'un titre 0 à la fois pour les anticorps anti-A et anti-B) 1ml du même plasma en 30 minutes soit avec 26 fois moins d'oligosaccharides par millilitre de plasma qu'avec l'utilisation du même oligosaccharide en solution,
- 50mg du support cellulosique de l'exemple 8B portant 0,287 mg d'oligosaccharides 6GrB1 neutralise 1ml du même plasma en 30 minutes soit avec 21 fois moins d'oligosaccharides par millilitre de plasma qu'avec l'utilisation du même oligosaccharide en solution,
- 200mg du support cellulosique de l'exemple 3 portant 3,28 mg d'oligosaccharides 4GrA5 neutralise 10ml du même plasma en 90 minutes soit avec 18 fois moins d'oligosaccharides par millilitre de plasma qu'avec l'utilisation du même oligosaccharide en solution,
- 200mg du support cellulosique de l'exemple 4 portant 2,6 mg d'oligosaccharides 4GrB5 neutralise 10ml du même plasma en 90 minutes soit avec 12 fois moins d'oligosaccharides par millilitre de plasma qu'avec l'utilisation du même oligosaccharide en solution.

Pour des durées d'incubations plus importantes, l'avantage des oligosaccharides fixés sur support est encore plus évident.

Par ailleurs, la neutralisation en phase liquide laisse subsister dans le plasma des complexes immuns et une quantité très importante d'oligosaccharide libres qui pourront avoir des effets secondaires néfastes pour les patients transfusés.

### I. Essais comparatifs avec des tri, tétra et hexa saccharides

Ces essais sont réalisés avec un plasma de titre 1/128 anti A et 1/8 anti B.

Les tests sont réalisés en gel (gel test).

| Exemple | Formule du produit testé | Résultats des tests en gel test |
|---|---|---|
| **Anticorps anti B** | | |
| Ex 10A | *6GrB1-Sp1-NH₂* | 0 |
| Ex 10B | *6GrB1-Sp1-NH₂ basse activation* | 0 |
| Ex 10C | *4GrB1-Sp1-NH₂* | 0 |
| Ex 10D | *6GrB1-Sp2-NH₂* | 0 |
| **Anticorps anti A** | | |
| Ex9A | *6GrA1-Sp1-NH₂* | 0 |
| Ex9B | *6GrA1-Sp1-NH₂* basse activation | 1/4 |
| Ex9C | *4GrA5-Sp1-NH₂* | 0 |
| Ex9D | *3GrA5-Sp1-NH₂* | 1/32 |
| Ex9E | *6GrA1-Sp2-NH₂* | 0 |
| Ex9F | *4GrA5-Sp2-NH₂* | 1/2 |
| Ex9G | *3GrA5-Sp2-NH₂* | 1/32 |

Conclusions :
Les résultats des tri-saccharides sont moins bons que ceux obtenus avec les autres oligosaccharides pour les configurations testées.

Il n'y a pas d'intérêt pour un bras PEG plus long : 8 versus 3 unités éthylène glycol.

Les résultats sont très proches entre 4GR5 et 6GR1.

### J. Test réalisés avec des gels de billes de polymère méthacrylique réduit (exemple comparatif 11) et non réduit (exemple 11) :

10 µL et 2 µL de chacun des deux gels obtenus aux exemples 11 et comparatif 11 sont incubés pendant une heure sous agitation rotative avec 1 mL de plasma de titre initial 1/1024 en anticorps anti-A.

Les résultats sont ensuite évalués par gel test :
Titre résiduel en anticorps anti-A après absorption :
1. Essais avec 10µL de gel / mL de plasma :

| | |
|---|---|
| Support non réduit | Support réduit |
| Ex. 11 | Exemple comparatif 11 |
| 1/4 | 1/8 |

2. Essais avec 2µL de gel / mL de plasma :

| | |
|---|---|
| Support non réduit | Support réduit |
| Ex. 11 | Exemple comparatif 11 |
| 1/128 | 1/256 |

Le test d'hémagglutination est négatif pour l'essai avec 10µL de gel non réduit/ml de plasma et positif pour les trois autres essais (gel réduit et non réduit à 2µL de gel/mL de plasma et gel réduit à 10µL de gel/mL de plasma). Comme dans le cas de l'utilisation d'un support du type cellulose, la présence de fonctions d'aldéhyde libres est favorable à une meilleure rétention des anticorps sur le support y compris pour les IgM comme l'atteste le résultat à 10µL de gel par mL de plasma. L'effet est toutefois moins marqué que sur la cellulose, du fait, sans doute, de la plus grande rigidité du support Tosoh par rapport aux fibres de cellulose et du plus faible nombre de fonctions aldéhydes libres sur la phase solide.

### K. Exemple sur sang total

15 ml de sang prélevé sur anticoagulant ACD (Acide citrate dextrose) contenant 5,5 ml de plasma sont mis en contact avec 55 mg du support de l'exemple 3 portant l'oligosaccharide 4GrA5, dans un tube de 50 mL et soumis à une agitation rotative de 100 rpm. Après une heure d'incubation à 22°C, le tube est centrifugé.

Après centrifugation, le support cellulosique occupe le fond du tube où il forme un culot blanc bien délimité surmonté par le culot d'hématies, puis par la couche leucoplaquettaire et enfin par le plasma.

Le titre anti A du plasma non traité contenu dans l'échantillon de sang total est de 1/128 en gel test, il présente une très forte agglutination en test d'hémagglutination.

Après traitement le titre du plasma en gel test est de 1/8, il est négatif en hémagglutination.

Cet exemple prouve que l'absorption des anticorps anti déterminant des groupes sanguins peut être réalisée sur sang total comme sur le plasma, avec une capacité de recueil des fractions sanguines équivalente à celle du sang non traité.

Cette absorption peut être réalisée directement dans une poche de prélèvement. Elle peut être mise en œuvre avant ou après déleucocytation.

### L- Evaluation des supports réduits et non réduits de l'Exemple 12 et de l'Exemple comparatif 12

Les supports de cellulose Ultrafine greffés par l'oligosaccharide **4GrA5-S-(CH₂)₂-NH₂** de l'exemple 12 (fonctions aldéhyde résiduelles libres) et de l'exemple comparatif 12 (fonctions aldéhyde résiduelles sous leur forme réduite) sont comparés.

Les tests sont réalisés avec 0,5 mL de plasma de titre 1/512 en anticorps anti A comme suit: 10 mg de support cellulosique greffé de l'exemple 12, d'une part et 10 mg de support de l'exemple comparatif 12, d'autre part, sont incubés pendant 60 min à 22°C sous agitation permanente en tube à hémolyse.

Le test d'agglutination direct est négatif pour le support non réduit et positif pour le support réduit, traduisant une moindre efficacité du support réduit.

En gel test, les résultats sont les suivants :

| Support testé | Titre anticorps anti A |
|---|---|
| Support non réduit de l'exemple 12 | 1/ 64 |
| Support réduit de l'exemple comparatif 12 | 1/ 128 |

La réduction du support s'accompagne d'une moindre efficacité pour l'élimination des anticorps. Dans ces conditions expérimentales, après épuration par le support réduit, il reste 25% des anticorps initiaux et seulement 12,5% avec le support portant des aldéhydes actifs.

### M- Exemple de purification des immunoglobulines thérapeutiques

Les immunoglobulines purifiées à partir de pool de plasmas humains constituent une fraction plasmatique d'un grand intérêt thérapeutique pour le traitement de pathologies auto-immunes et pour la réduction des risques infectieux chez les patients immunodéprimés.

Ces anticorps purifiés, commercialisés sous le terme générique d'IVIG (Intravenous immunoglobulins), représentent la fraction IgG totale des protéines plasmatiques. Ils contiennent des anticorps anti groupes sanguins A et B à un titre suffisamment élevé pour présenter des risques hémolytiques chez les patients multi-injectés. L'augmentation du rendement de purification de cette fraction IgG du plasma et l'évolution du profil des donneurs rendent l'épuration anti A et B indispensable.

Les supports préparés dans l'exemple 12 ont été utilisés pour une telle purification. Les essais ont été conduits avec un lot d'IVIG commercial (PRIVIGEN de CSL-Behring). Les titres par gel test de cette préparation à 10% d'immunoglobulines étaient respectivement de 1/128 en anti A et 1/32 en anti B.

### Premier essai

### Conditions de l'essai :

5 mg de support A et 5 mg de support B préparés à l'exemple 12 ont été incubés 30 min avec 200 µL de la préparation commerciale non diluée. Après centrifugation, le titre du surnageant a été mesuré.

### Résultats :

Les titres par gel test de cette préparation à 10% d'immunoglobulines après absorption étaient respectivement de 1/16 en anti A et 1/4 en anti B, soit une élimination de 87,5 % des anticorps anti A et anti B.

### Deuxième essai

### Conditions de l'essai :

50 mg de support A et 50 mg de support B préparés à l'exemple 12 sont réunis dans une colonne (Colonne 4g Telos UK). Un volume de 10 mL de la solution d'immunoglobuline diluée 1/5 en solution physiologique (PBS) est passé sur la colonne en 30 min à 22°C.

Les titres anti A et anti B du filtrat ont été mesurés.

### Résultats :

Les titres par gel test de cette préparation à 2% d'immunoglobulines après absorption étaient respectivement de 1/1 en anti A et 1/1 en anti B, soit une élimination de plus de 95 % des anticorps anti A et anti B.

Les supports préparés selon l'exemple 12 sont donc parfaitement efficaces pour la purification des préparations immunoglobulines.

Cette purification (élimination des anticorps anti groupes sanguins A et B) peut être réalisée en colonne avec des résultats comparables à ceux obtenus en phase liquide avec le support en suspension sous agitation.

### N- Neutralité biologique des supports

Les deux supports préparés à l'exemple 12 ont été testés pour leur spécificité, afin de montrer que seuls les anticorps anti groupes sanguins A et B sont éliminés des préparations d'immunoglobulines thérapeutiques.

Les tests ont été conduits sur la phase solide seule (cellulose Ultrafine activée de l'exemple 12) et sur les deux Supports A et B greffés avec les oligosaccharides correspondants du même exemple.

La solution d'immunoglobuline (IVIG) utilisée au paragraphe M précédent a été traitée, après dilution à 2% en PBS, dans les conditions suivantes :
1-Test phase solide seule (cellulose activée) :
   50 mg de cellulose activée dans 1 mL d'IVIG 2%. Incubation pendant une heure à 22°C, centrifugation, filtration.
   Mesure sur le filtrat de la concentration protéique totale et des titres anti A et anti B. La concentration protéique totale des immunoglobulines est mesurée sur l'appareil Biophotometer plus de la société Eppendorf (D) à 260, 280 et 340 nm. Les résultats sont ajustés pour les immunoglobulines selon le logiciel du fabricant. Le titre est mesuré par le Gel test (Biorad CH).
2-Test sur les Supports finis :
   10 mg de Support A et 10 mg de Support B dans 1 mL d'IVIG 2%. Incubation pendant une heure à 22°C, centrifugation, filtration.
   Mesure sur le filtrat de la concentration protéique totale et des titres anti A et anti B.

### Résultats :

1-Phase solide seule

| | Concentration protéique | titre anti A | titre anti B |
|---|---|---|---|
| Test avant traitement | 17,41 mg/mL +/-0,27 | 1/32 | 1/16 |
| Test après traitement | 17,40 mg/mL +/-0,26 | 1/32 | 1/16 |

2-Test sur Support A + Support B

| | Concentration protéique | titre anti A | titre anti B |
|---|---|---|---|
| Test avant traitement | 18,50 mg/mL +/-0,28 | 1/32 | 1/16 |
| Test après traitement | 18,40 mg/mL +/-0,27 | 0 | 0 |

Le premier test indique que la phase solide seule, avec 1 fonction aldéhyde pour moins de 18 glucoses et une forte surcharge en cellulose (X5), ne fixe pas d'immunoglobuline par elle-même.

Le second test montre que la légère réduction de concentration protéique est cohérente avec l'élimination spécifique des anticorps anti A et anti B et seulement de ces anticorps.

Les supports oligosaccharidiques ainsi définis avec des fonctions aldéhyde libres en large excès par rapport à la concentration d'antigènes oligosaccharidiques greffés sont parfaitement adaptés à la purification des immunoglobulines plasmatiques.

## Revendications

1. - Support pour la purification d'un liquide biologique comprenant une phase solide sur laquelle sont greffées, par liaison covalente, des molécules d'au moins un oligosaccharide capable de se lier à un ou plusieurs anticorps, ladite phase solide étant porteuse de fonctions aldéhyde -CHO libres, lesdites fonctions aldéhyde libres -CHO étant présentes directement sur la phase solide et non sur les molécules d'oligosaccharide(s).

2. - Support selon la revendication 1 **caractérisé en ce que** le liquide biologique est du sang ou un produit issu du sang, et ledit au moins un oligosaccharide est capable de se lier aux anticorps anti déterminant A, B ou H des groupes sanguins.

3. - Support selon l'une quelconque des revendications 1 à 2 **caractérisé en ce que** le ratio nombre de molécules d'oligosaccharide(s) greffées sur la phase solide / nombre de fonctions aldéhyde libres présentes sur la phase solide appartient à la gamme allant de 1/400 à 1/1, de préférence à la gamme allant de 1/200 à 1/10.

4. - Support selon l'une quelconque des revendications 1 à 3 **caractérisé en ce que** le support comporte de 1 à 100 mg d'oligosaccharide(s) /g de support, préférentiellement entre 2 et 40 mg d'oligosaccharide(s) /g de support.

5. - Support selon l'une quelconque des revendications 1 à 4 **caractérisé en ce que** les fonctions aldéhyde libres assurent une stabilisation des anticorps, consécutivement à leur reconnaissance immunologique par lesdites molécules d'oligosaccharide(s) greffées sur la phase solide, notamment par liaison covalente, d'une asparagine, lysine, arginine, glutamine, tryptophane, tyrosine, ou cystéine, présent sur l'anticorps sur une fonction aldéhyde libre présente sur la phase solide.

6. - Support selon l'une quelconque des revendications 1 à 5 **caractérisé en ce que** les molécules d'oligosaccharide(s) sont greffées sur la phase solide par l'intermédiaire d'une liaison résultant de la réaction d'une fonction amine du type -NH₂, -NH-NH₂ ou -O-NH₂ portée avant greffage par l'oligosaccharide et d'une fonction aldéhyde -CHO portée avant greffage par la phase solide.

7. - Support selon l'une quelconque des revendications 1 à 6 **caractérisé en ce que** la phase solide est un polysaccharide et les fonctions aldéhyde libres résultent d'une oxydation périodique.

8. - Support selon l'une quelconque des revendications 1 à 7 **caractérisé en ce que** la phase solide est de la cellulose.

9. - Support selon la revendication 8 **caractérisé en ce que** la cellulose greffée comporte des unités de formule :
-CH(CHO)-CH(CH₂OH)-O-CH(CH₂-NH-**L**-oligo)-O-
et/ou des unités de formule :
-CH(CH₂-NH-**L**-oligo)-CH(CH₂OH)-O-CH(CHO)-O-
dans lesquelles **L** est un bras de liaison et **oligo** est un oligosaccharide capable de se lier à un ou plusieurs anticorps, de préférence un oligosaccharide capable de se lier aux anticorps anti déterminant A, B ou H des groupes sanguins.

10. - Support selon l'une quelconque des revendications 1 à 9 **caractérisé en ce que** le greffage des molécules d'oligosaccharide(s) sur la phase solide est réalisé par l'intermédiaire d'un enchaînement -NH-**L**- dans le sens phase solide oligosaccharide, **L** étant un bras de liaison.

11. - Support selon la revendication 9 ou 10 **caractérisé en ce que** le bras de liaison **L** comporte un groupe triazole ou une liaison -S- et/ou une chaîne polyéthylène glycol et/ou une ou plusieurs chaînes alkylène identiques ou différentes du type -(CH₂)ₜ- avec t qui peut être 2, 3, 4 ou 5 notamment.

12. - Support selon la revendication 9 ou 10 **caractérisé en ce que** le bras de liaison **L** correspond à l'enchaînement :
-CH₂-CH₂-S-CH₂-CH₂-CH₂-N(COCH₃)-, le groupe N(COCH₃) étant directement lié à une unité saccharidique de l'oligosaccharide, de préférence sur le carbone anomérique de ladite unité saccharidique.

13. - Support selon l'une quelconque des revendications 1 à 12 **caractérisé en ce que** l'oligosaccharide est un trisaccharide, un tetrasaccharide, un pentasaccharide ou un hexasaccharide, capable de se lier aux anticorps anti déterminant B ou aux anticorps anti déterminant A.

14. - Support selon l'une quelconque des revendications 1 à 13 **caractérisé en ce que** l'oligosaccharide est choisi parmi GalNAcα1-3(Fucα1-2)Galβ1, Galα1-3(Fucα1-2)Galβ1, GalNAcα1-3(Fucα1-2)Galβ1-4Glc, Galα1-3(Fucα1-2)Galβ1-4Glc, GalNAcα1-3(Fucα1-2)Galα1-3GlcNAcβ1-3Galβ1-4Glc, Galα1-3(Fucα1-2)Galβ1-3GlcNAcβ1-3Galβ1-4Glc, GalNAcα1-3(Fucα1-2)Galβ1-4GlcNAcβ1-3Galβ1-4Glc, et Galα1-3(Fucα1-2)Galβ1-4GlcNAcβ1-3Galβ1-4Glc, Fucα1-2Galβ1-3GlcNAc, Fucα1-2Galβ1-4GlcNAc, Fucα1-2Galβ1-4Glc, Fucα1-2Galβ1-3GlcNAcβ1-3Gal, Fucα1-2Galβ1-4GlcNAcβ1-3Gal, Fucα1-2Galβ1-3GalNAcβ1-3Gal, Fucα1-2Galβ1-3GlcNAcβ1-3Galβ1-4Glc, Fucα1-2Galβ1-4GlcNAcβ1-3Galβ1-4Glc, les oligosaccharides GalNAcα1-3(Fucα1-2)Galβ1-4Glc et Galα1-3(Fucα1-2)Galβ1-4Glc étant préférés.

15. - Procédé de purification d'un liquide biologique comprenant des anticorps, dans lequel le liquide biologique est mis en contact avec au moins un support tel que défini à l'une quelconque des revendications 1 à 14, de manière à obtenir la capture d'anticorps présents dans ledit liquide biologique par liaison(s) avec au moins certaines des molécules d'oligosaccharide(s) greffées sur la phase solide.

16. - Procédé de préparation d'un support conforme à l'une quelconque des revendications 1 à 14, dans lequel le greffage des molécules d'oligosaccharide(s) est réalisé sur une phase solide porteuse de fonctions aldéhyde -CHO libres, par réaction, sur une partie seulement des fonctions aldéhyde -CHO libres présentes sur la phase solide, d'un oligosaccharide porteur d'une fonction réactive -NH₂, -NH-NH₂ ou -O-NH₂, éventuellement sous forme protégée, et d'une fonction aldéhyde -CHO portée avant greffage par la phase solide, en présence d'un réducteur qui ne réduit pas les fonctions aldéhyde libres ne réagissant pas avec les fonctions amine, tel que le cyanoborohydrure de sodium.

17. - Utilisation d'au moins un support tel que défini à l'une quelconque des revendications 1 à 14 pour la purification d'un liquide biologique contenant des anticorps à capturer, dans lequel ledit liquide biologique est mis en contact avec le au moins dit support pour assurer une stabilisation de la liaison des anticorps IgG, qui viennent se lier à au moins certaines des molécules d'oligosaccharide(s) greffées à la surface de la phase solide.

## Patentansprüche

1. Träger zur Reinigung einer biologischen Flüssigkeit, umfassend eine Festphase, auf die durch kovalente Bindung Moleküle wenigstens eines Oligosaccharids aufgepfropft sind, das in der Lage ist, an einen oder mehrere Antikörper zu binden, wobei die Festphase freie -CHO Aldehydfunktionen trägt, wobei die freien -CHO Aldehydfunktionen direkt auf der Festphase und nicht auf den Oligosaccharid(en)-Molekülen vorhanden sind.

2. Träger nach Anspruch 1, **dadurch gekennzeichnet, dass** die biologische Flüssigkeit Blut oder ein aus Blut stammendes Produkt ist und dass das wenigstens eine Oligosaccharid in der Lage ist, an die Anti-Determinante-Antikörper A, B oder H der Blutgruppen zu binden.

3. Träger nach einem der Ansprüche 1 bis 2, **dadurch gekennzeichnet, dass** das Verhältnis der Anzahl der auf die Festphase gepfropften Oligosaccharid(en)-Moleküle zur Anzahl der auf der Festphase vorhandenen freien Aldehydfunktionen im Bereich von 1/400 bis 1/1, vorzugsweise im Bereich von 1/200 bis 1/10 liegt.

4. Träger nach einem der Ansprüche 1 bis 3, **dadurch gekennzeichnet, dass** der Träger 1 bis 100 mg Oligosaccharid(e)/g Träger, vorzugsweise zwischen 2 und 40 mg Oligosaccharid(e)/g Träger enthält.

5. Träger nach einem der Ansprüche 1 bis 4, **dadurch gekennzeichnet, dass** die freien Aldehydfunktionen eine Stabilisierung der Antikörper nach ihrer immunologischen Erkennung durch die auf die Festphase aufgepfropften Oligosaccharid(en)-Moleküle, insbesondere durch kovalente Bindung, eines Asparagins, Lysins, Arginins, Glutamins, Tryptophans, Tyrosins oder Cysteins, das auf dem Antikörper vorhanden ist, auf einer auf der Festphase vorhandenen freien Aldehydfunktion gewährleisten.

6. Träger nach einem der Ansprüche 1 bis 5, **dadurch gekennzeichnet, dass** die Oligosaccharid(en)-Moleküle mittels einer Bindung, die aus der Reaktion einer Aminfunktion vom Typ -NH₂, -NH-NH₂ oder -O-NH₂, welche vor dem Pfropfen durch das Oligosaccharid getragen wird, und einer -CHO Aldehydfunktion, welche vor dem Pfropfen durch die Festphase getragen wird, auf die Festphase aufgepfropft sind.

7. Träger nach einem der Ansprüche 1 bis 6, **dadurch gekennzeichnet, dass** die Festphase ein Polysaccharid ist und die freien Aldehydfunktionen aus einer periodischen Oxidation resultieren.

8. Träger nach einem der Ansprüche 1 bis 7, **dadurch gekennzeichnet, dass** die Festphase Cellulose ist.

9. Träger nach Anspruch 8, **dadurch gekennzeichnet, dass** die gepfropfte Cellulose Einheiten enthält mit der Formel:
-CH(CHO)-CH(CH₂OH)-O-CH(CH₂-NH-L-oligo)-O-
und/oder Einheiten mit der Formel:
-CH(CH₂-NH-L-oligo)-CH(CH₂OH)-O-CH(CHO)-O-
bei denen L ein Bindungsarm ist und oligo ein Oligosaccharid ist, das in der Lage ist, an einen oder mehrere Antikörper zu binden, vorzugsweise ein Oligosaccharid, das in der Lage ist, an die Anti-Determinante-Antikörper A, B oder H der Blutgruppen zu binden.

10. Träger nach einem der Ansprüche 1 bis 9, **dadurch gekennzeichnet, dass** das Pfropfen der Oligosaccharid(en)-Moleküle auf die Festphase mittels einer -NH-L-Verknüpfung in Richtung Festphase Oligosaccharid erfolgt, wobei L ein Bindungsarm ist.

11. Träger nach Anspruch 9 oder 10, **dadurch gekennzeichnet, dass** der Bindungsarm L eine Triazolgruppe oder eine -S- Bindung und/oder eine Polyethylenglykolkette und/oder eine oder mehrere Alkylenketten, identische oder unterschiedliche, vom Typ -(CH₂)ₜ- umfasst, wobei t insbesondere 2, 3, 4 oder 5 sein kann.

12. Träger nach Anspruch 9 oder 10, **dadurch gekennzeichnet, dass** der Bindungsarm L der Verknüpfung entspricht:
-CH₂-CH₂-S-CH₂-CH₂-CH₂-N(COCH₃)-, wobei die Gruppe N(COCH₃) direkt an eine Saccharideinheit des Oligosaccharids gebunden ist, vorzugsweise an den anomeren Kohlenstoff der Saccharideinheit.

13. Träger nach einem der Ansprüche 1 bis 12, **dadurch gekennzeichnet, dass** das Oligosaccharid ein Trisaccharid, ein Tetrasaccharid, ein Pentasaccharid oder ein Hexasaccharid ist, das in der Lage ist, an die Anti-Determinante-Antikörper B oder an die Anti-Determinante-Antikörper A zu binden.

14. Träger nach einem der Ansprüche 1 bis 13, **dadurch gekennzeichnet, dass** das Oligosaccharid ausgewählt ist aus GalNAcα1-3(Fucα1-2)Galβ1, Galα1-3(Fucα1-2)Galβ1, GalNAcα1-3(Fucα1-2)Galβ1-4Glc, Galα1-3(Fucα1-2)Galβ1-4G1c, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, GalNAcα1-3(Fucα1-2)Galβ1-4GlcNAcβ1-3Galβ1-4Glc, und Galα1-3(Fucα1-2)Galβ1-4GlcNAcβ1-3Galβ1-4Glc, Fucα1-2Galβ1-3GlcNAc, Fucα1-2Galβ1 - 4GlcNAc, Fucα1-2Galβ1-4G1c, Fucα1-2Galβ1-3GlcNAcβ1-3Gal, Fucα1-2Galβ1-4GlcNAcβ1-3Gal, Fucα1-2Galβ1 -3GalNAcβ1-3Gal, Fucα1-2Galβ1 -3GlcNAcβ1-3Galβ1-4Glc, Fucα1-2Galβ1-4GlcNAcβ1-3Galβ1-4Glc, wobei die Oligosaccharide GalNAcα1-3(Fucα1-2)Galβ1-4Glc und Galα1-3(Fucα1-2)Galβ1-4Glc bevorzugt sind.

15. Verfahren zur Reinigung einer biologischen Flüssigkeit, die Antikörper umfasst, wobei die biologische Flüssigkeit mit wenigstens einem Träger, wie er in einem der Ansprüche 1 bis 14 definiert ist, in Kontakt gebracht wird, um das Einfangen von Antikörpern, welche in der biologischen Flüssigkeit vorhanden sind, durch Bindung(en) mit wenigstens einigen der auf die Festphase gepfropften Oligosaccharid(en)-Moleküle zu erreichen.

16. Verfahren zur Herstellung eines Trägers nach einem der Ansprüche 1 bis 14, bei dem das Aufpropfen der Oligosaccharid(en)-Moleküle auf einer festen Phase, die freie -CHO Aldehydfunktionen trägt, durch Reaktion - auf nur einem Teil der freien - CHO Aldehydfunktionen, welche auf der Festphase vorhanden sind - eines Oligosaccharids, das eine reaktive -NH₂, -NH-NH₂ oder -O-NH₂-Funktion trägt, eventuell in geschützter Form, und einer -CHO Aldehydfunktion, die vor dem Pfropfen durch die Festphase getragen wird, in Gegenwart eines Reduktionsmittels, das die freien Aldehydfunktionen, die nicht mit den Aminfunktionen reagieren, nicht reduziert, wie Natriumcyanoborhydrid, vollzogen wird.

17. Verwendung wenigstens eines Trägers wie er in einem der Ansprüche 1 bis 14 definiert ist, zur Reinigung einer biologischen Flüssigkeit, die einzufangende Antikörper enthält, wobei die biologische Flüssigkeit mit dem wenigstens einen Träger in Kontakt gebracht wird, um eine Stabilisierung der Bindung der IgG-Antikörper, die an wenigstens einige der auf die Oberfläche der Festphase gepfropften Oligosaccharid(en)-Moleküle binden, sicherzustellen.

## Claims

1. A substrate for purifying a biological liquid comprising a solid phase onto which molecules of at least one oligosaccharide that is capable of binding to one or more antibodies are grafted by covalent bonding, said solid phase carrying free aldehyde functions -CHO, said free aldehyde functions -CHO being present directly on the solid phase, and not on the oligosaccharide molecules.

2. A substrate according to claim 1, **characterized in that** the biological liquid is blood or a product obtained from blood, and said at least one oligosaccharide is capable of binding to anti-determinant A, B, or H antibodies of blood groups.

3. A substrate according to any one of claims 1 to 2, **characterized in that** the ratio of the number of molecules of oligosaccharide(s) grafted onto the solid phase/number of free aldehyde functions present on the solid phase is in the range 1/400 to 1/1, preferably in the range 1/200 to 1/10.

4. A substrate according to any one of claims 1 to 3, **characterized in that** the substrate comprises 1 to 100 mg of oligosaccharide(s)/g of substrate, preferably between 2 and 40 mg of oligosaccharide(s)/g of substrate.

5. A substrate according to any one of claims 1 to 4, **characterized in that** the free aldehyde functions stabilize the antibodies following their immunological recognition by said molecules of oligosaccharide(s) grafted onto the solid phase, in particular by covalent bonding of an asparagine, lysine, arginine, glutamine, tryptophan, tyrosine, or cysteine, present on the antibody onto a free aldehyde function present on the solid phase.

6. A substrate according to any one of claims 1 to 5, **characterized in that** the molecules of oligosaccharide(s) are grafted onto the solid phase via a bond resulting from the reaction of an amine function of the type -NH₂, -NH-NH₂, or -O-NH₂ carried by the oligosaccharide before grafting and an aldehyde function -CHO carried by the solid phase before grafting.

7. A substrate according to any one of claims 1 to 6, **characterized in that** the solid phase is a polysaccharide and the free aldehyde functions result from a periodic oxidation.

8. A substrate according to any one of claims 1 to 7, **characterized in that** the solid phase is cellulose.

9. A substrate according to claim 8, **characterized in that** the grafted cellulose comprises units with formula:
-CH(-CHO)-CH(CH2OH)-O-CH(CH2-NH-**L**-oligo)-O- and
/or units with formula:
-CH(CH2-NH-**L**-oligo)-CH(CH2OH)-O-CH(-CHO)-O-
in which **L** is a spacer arm and **oligo** is an oligosaccharide that is capable of binding to one or more antibodies, preferably an oligosaccharide that is capable of binding to anti-determinant A, B or H antibodies of blood groups.

10. A substrate according to any one of claims 1 to 9, **characterized in that** the molecules of oligosaccharide are grafted onto the solid phase via a concatenation -NH-**L**- in the solid phase-oligosaccharide direction, **L** being a spacer arm.

11. A substrate according to claim 9 or claim 10, **characterized in that** the spacer arm **L** comprises a triazole group or a -S- linker and/or a polyethylene glycol chain and/or one or more identical or different alkylene chains of the type -(CH₂)ₜ- in which t may be 2, 3, 4, or 5 in particular.

12. A substrate according to claim 9 or claim 10, **characterized in that** the spacer arm **L** corresponds to the concatenation:
-CH₂-CH₂-S-CH₂-CH₂-CH₂-N(COCH₃)-,
the group N(COCH₃) being bonded directly to a saccharide unit of the oligosaccharide, preferably onto the anomeric carbon of said saccharide unit.

13. A substrate according to any one of claims 1 to 12, **characterized in that** the oligosaccharide is a trisaccharide, a tetrasaccharide, a pentasaccharide or a hexasaccharide, capable of binding to anti-determinant B antibodies or the oligosaccharide is capable of binding to anti-determinant A antibodies.

14. A substrate according to any one of claims 1 to 13, **characterized in that** the oligosaccharide is selected from GalNAcα1-3(Fucα1-2)Galβ1, Galα1-3(Fucα1-2)Galβ1, GalNAcα1-3(Fucα1-2)Galβ1-4Glc, Galα1-3(Fucα1-2)Galβ1-4Glc, GalNAcα1-3(Fucα1-2)Galβ1-3GlcNAcβ1-3Galβ1-4Glc, Galα1-3(Fucα1-2)Galβ1-3GlcNAcβ1-3Galβ1-4Glc, GalNAcα1-3(Fucα1-2)Galβ1-4GlcNAcβ1-3Galβ1-4Glc, and Galα1-3(Fucα1-2)Galβ1-4GlcNAcβ1-3Galβ1-4Glc, Fucα1-2Galβ1-3GlcNAc, Fucα1-2Galβ1-4GlcNAc, Fucα1-2Galβ1-4Glc, Fucα1-2Galβ1-3GlcNAcβ1-3Gal, Fucα1-2Galβ1-4GlcNAcβ1-3Gal, Fucα1-2Galβ1-3GalNAcβ1-3Gal, Fucα1-2Galβ1-3GlcNAcβ1-3Galβ1-4Glc, Fucα1-2Galβ1-4GlcNAcβ1-3Galβ1-4Glc, the oligosaccharides GalNAcα1-3(Fucα1-2)Galβ1-4Glc and Galα1-3(Fucα1-2)Galβ1-4Glc being preferred.

15. A purification method for purifying a biologic liquid including antibodies, in which the biological liquid is brought into contact with at least one substrate as defined in any one of claims 1 to 14, in a manner such as to obtain the capture of antibodies present in said biological liquid by binding with at least some of the molecules of oligosaccharide(s) grafted onto said solid phase.

16. A preparation method for preparing a substrate in accordance with any one of claims 1 to 14, in which the grafting of the molecules of oligosaccharide(s) is carried out onto a solid phase carrying free aldehyde functions -CHO by reaction on only a portion of the free aldehyde functions -CHO present on the solid phase, of an oligosaccharide carrying a reactive function -NH₂, -NH-NH₂, or -O-NH₂, optionally in a protected form, and an aldehyde function -CHO carried by the solid phase before grafting, in the presence of a reducing agent that does not reduce the free aldehyde functions not reacting with the amine functions, such as sodium cyanoborohydride.

17. Use of at least one substrate as defined in any one of claims 1 to 14 to purify a biological liquid containing antibodies to be captured, in which said biological liquid is brought into contact with the at least said substrate in order to ensure stabilization of the bond to the antibodies IgG, which bind to at least certain of the molecules of oligosaccharide(s) grafted to the surface of the solid phase.
